(19) Europäisches Patentamt

European Patent Office

Office européen des brevets

(11) **EP 4 371 586 B1**

## (12) EUROPEAN PATENT SPECIFICATION

(45) Date of publication and mention
of the grant of the patent:
**16.04.2025 Bulletin 2025/16**

(21) Application number: **23174685.0**

(22) Date of filing: **22.05.2023**

(51) International Patent Classification (IPC):
**A61L 27/46** (2006.01)    **A61L 27/54** (2006.01)
**C07K 7/06** (2006.01)    **C07K 17/00** (2006.01)

(52) Cooperative Patent Classification (CPC):
(C-Sets available)
**A61L 27/54; A61L 27/46; C07K 7/06; C07K 17/00;**
A61L 2300/25; A61L 2300/252; A61L 2300/412;
A61L 2430/02    (Cont.)

(54) **MULTIFUNCTIONAL CHITOSAN COMPOSITES FOR BONE DEFECT FILLING AND BONE TISSUE REGENERATION AND METHODS FOR OBTAINING THEM**

MULTIFUNKTIONALE CHITOSAN-KOMPOSITEN FÜR DIE FÜLLUNG VON KNOCHENDEFEKTEN UND DIE REGENERIERUNG VON KNOCHENGEWEBE SOWIE VERFAHREN ZU DEREN HERSTELLUNG

COMPOSITES DE CHITOSANE MULTIFONCTIONNEL POUR LE COMBLEMENT DE DÉFAUTS OSSEUX ET LA RÉGÉNÉRATION DE TISSUS OSSEUX ET PROCÉDÉS POUR LEUR OBTENTION

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **18.11.2022 PL 44287823**
**18.11.2022 PL 44288023**

(43) Date of publication of application:
**22.05.2024 Bulletin 2024/21**

(73) Proprietors:
• **Siec Badawcza Lukasiewicz**
**- Instytut Ceramiki i Materialow Budowlanych**
**31-983 Krakow (PL)**
• **Uniwersytet Gdanski**
**80-309 Gdansk (PL)**
• **Politechnika Wroclawska**
**50-370 Wroclaw (PL)**
• **Instytut Biotechnologii I Medycyny Molekularnej**
**80-180 Gdansk (PL)**
• **SENSDX SA**
**02-676 Warszawa (PL)**

(72) Inventors:
• **Biernat, Monika**
**Warszawa (PL)**
• **Ciolek, Lidia**
**Warszawa (PL)**

• **Wozniak, Anna**
**Warszawa (PL)**
• **Jaegermann, Zbigniew**
**Spychowo (PL)**
• **Kubis, Agnieszka**
**Gdansk (PL)**
• **Karska, Natalia**
**Gdynia (PL)**
• **Sawicka, Justyna**
**Gdansk (PL)**
• **Rodziewicz-Motowidlo, Sylwia**
**83-010 Rotmanka (PL)**
• **Chraniuk, Milena**
**Ostroda (PL)**
• **Gromadzka, Beata**
**Gdynia (PL)**
• **Panasiuk, Miroslawa**
**Gdynia (PL)**
• **Bollin, Piotr**
**Rotmanka (PL)**

(74) Representative: **Czarnik, Maciej**
**Triloka Czarnik Ozóg Kancelaria Patentowa i Adwokacka sp.p.**
**ul. Kornela Ujejskiego 12/7**
**30-102 Kraków (PL)**

(56) References cited:
**CN-A- 113 134 114**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

• MONIKA BIERNAT ET AL: "Porous chitosan/ZnO-doped bioglass composites as carriers of bioactive peptides", INTERNATIONAL JOURNAL OF APPLIED CERAMIC TECHNOLOGY, BLACKWELL PUBLISHING, MALDEN, MA, US, vol. 17, no. 6, 26 August 2020 (2020-08-26), pages 2807 - 2816, XP072313742, ISSN: 1546-542X, DOI: 10.1111/IJAC.13609
• CHEN YU-CHEN ET AL: "Bioactive pseudopeptidic analogues and cyclostereoisomers of osteogenic growth peptide C-terminal pentapeptide, OGP(10-14)", JOURNAL OF MEDICINAL CHEMISTRY, AMERICAN CHEMICAL SOCIETY, US, vol. 45, no. 8, 11 April 2002 (2002-04-11), pages 1624 - 1632, XP002599698, ISSN: 0022-2623, [retrieved on 20020315], DOI: 10.1021/JM0104791

Remarks:
The complete document including Reference Table(s) and the Sequence Listing(s) can be downloaded from the EPO website

(52) Cooperative Patent Classification (CPC): (Cont.)

C-Sets
A61L 27/46, C08L 5/08

**Description**

The invention is defined in the claims. The present disclosure refers to porous multifunctional composite for use as a biocompatible material for filling bone defects and regenerating bone tissue, in which the polymer matrix is chitosan, the filler is bioglass and the whole is enriched with a biologically active, engineered peptide with a specific amino acid sequence, with proven pro-regenerative properties - referred to in the patent description as "ug4" and/or a biologically active, engineered peptide with a specific amino acid sequence, with proven antimicrobial and anti-inflammatory properties - referred to in the patent description as "ug46", or is enriched with this peptide in the form of fibrils with proven antimicrobial properties - referred to in the patent description as "UG46". The subject of the invention is also a method of obtaining the composite.

The invention finds application as a porous material for use as a biocompatible material for filling bone defects and/or regenerating bone tissue. Compositions and methods not comprising the peptide sequence referred to as "ug46" or SEQ3 are not part of the invention.

Biocomposites for bone tissue reconstruction should be biocompatible, nontoxic, non-carcinogenic and non-allergenic materials. Due to their place of application, they have high requirements as described in ISO 10993 standards. In the publication "Bone grafts: which is the ideal biomaterial?" [J. Clin. Periodontol. 2019, 46, 92- 102] presents the direction that biomaterials should support the restoration of damaged tissues and that the implants should undergo controlled degradation. In the case of bone tissue implants, a great deal of attention is paid to their osteoconductive abilities and porosity. It is assumed that the pore layout and size should allow good cell migration and ensure easy delivery of nutrients within the implant. The optimal average pore size of implants for bone tissue cells should be in the range 50-400 $\mu$m "Design of artificial extracellular matrices for tissue engineering" [Prog Polym Sci. 2011;36:238-268]. Relevant there are also smaller pores that facilitate vascularisation, while pores with a diameter of more than 500 $\mu$m promote fibrous tissue formation. The prevention of post- implant bacterial infections is also receiving much attention in ongoing research, e.g. through the incorporation of bioglasses with antimicrobial activity into the composition of biomaterials 'Mesoporous bioactive glasses: Promising platforms for antibacterial strategies' [Acta Biomat. 2018, 81, 1-19], "Controlling the microstructure of lyophilised porous biocomposites by the addition of ZnO-doped bioglass" [Int. J. Appl. Ceram. Techn. 2017, 14, 1107-16].

There is information in the literature on the results of composite materials research, with a great deal of attention paid to bioactive glasses "Bioactivity and osteoinductivity of glasses and glassceramics and their material determinants" [Ceram. Int. 2016, 42, 14313-25] and composites based on biodegradable polymers "A Review of Bioactive Glass/Natural Polymer Composites: State of the Art' [Mater. 2020, 13, 1-38], as well as 'Polymeric biomaterials' [Acta Mater. 2000, 48, 263-277]. One of the natural polymers used to create composites is chitosan, which is obtained by chitin deacetylation and contains linearly linked $\beta$-1,4-glycosidic bonds of D-glucosamine and N-acetyl-D- glucosamine molecules. It has high biocompatibility, antimicrobial and hemostatic properties and is biodegradable 'Chitosan: A versatile biopolymer for orthopaedic tissue- engineering' [Biomaterials 2005;26: 5983-5990]. Its osteoconductive abilities make it useful in hard tissue engineering "Prospects of chitosan-based scaffolds for growth factor release in tissue engineering" [Int J Biol Macromol, Part B. 2016;93:1382-1389], however, its mechanical properties and biological activity must be enhanced with e.g. bioglasses "3D Nanocomposite chitosan/bioactive glass scaffolds obtained using two different routes: an evaluation of the porous structure and mechanical properties" [Quim. Nova, Vol. 39, No. 4, 462-466, 2016]. Bioactive glasses are a component of composites that can induce bioactivity, osteointegration and bactericidal activity of composites. In particular, bioglasses produced by the sol-gel method show high bioactivity, i.e. the ability to form an apatite interlayer "Molecular Control of Bioactivity in Sol-Gel Glasses" [J. Sol-Gel Sci. Tech. 1998, 13, 245-50]. The appropriate chemical composition of the glasses influences the qualitative composition of the released ions, which then play an important role in the healing process around the implant and bone regeneration "The synergistic effects of Sr and Si bioactive ions on osteogenesis, osteoclastogenesis and angiogenesis for osteoporotic bone regeneration" [Acta Biomat. 2017, 61, 217-232]. Modification of the composition of bioglasses by partially replacing CaO by MgO or SrO increases the surface reactivity, and this during contact between the biomaterial and body fluid increases the ability to form an apatite interlayer. Sr/Ca substitution is an established strategy for creating biomaterials for use in bone regeneration therapy 'The effects of strontium-substituted bioactive glasses on osteoblasts and osteoclasts in vitro' [Biomat. 2010, 31, 3949-56]. When metal ions or oxides with bactericidal capabilities are introduced into the chemical composition of glasses, antibacterial materials are obtained. Various substances have been used as bactericidal agents in composites "Graphene oxide, chitosan and silver nanocomposite as a highly effective antibacterial agent against pathogenic strains" [Coll. Surfaces A, 2018, 13, 245-50].

Bactericidal activity is also characterised by ions of metals, and bioactive glasses containing them are a potential alternative for systemic delivery of antibiotics to prevent bacterial infections: "Glasses in bone regeneration: a multiscale issue" [J. Non-Cryst. Solids 432 (2016) 9-14], "Mesoporous silica-based bioactive glasses for antibiotic-free antibacterial applications" [Mater. Sci. Eng. C 2018, 83, 99-107].

A hydrogel composite based on alginate-chitosan/admixed by calcium phosphate/ bioglass is described in 'Preparation and characterisation of trace elements-multidoped injectable biomimetic materials for minimally invasive treatment of osteoporotic bone trauma' [J Biomed Mat Res A, 2010, 95A(4), 1170-81], however, calcium phosphate doped with ZnO and/or SrO and a bioglass composition of 58% $SiO_2$ - 36% CaO - 6% $P_2O_5$ is introduced as filler, and the entire system is not porous, but exists in gel form.

The inconvenience of this solution is the gel-like form of the material, which does not provide an environment for cell migration after implantation of the composite. Migration is only made possible by the porous form of the material, which has an adequate pore size.

A method for preparing porous chitosan scaffolds has been described in the literature, among others, using the example of a chitosan-agarose-based scaffold. The method involved preparing a solution of chitosan in an aqueous solution of acetic acid and an aqueous solution of agarose at 90 °C and then, after adding the chitosan solution to the agarose solution, freezing the whole at -80 °C and freeze-drying. The resulting material was used in cartilage tissue engineering (Society for Biomaterials, 2017,105(7),1845-1855).

Experience in methods of obtaining porous polymer scaffolds enriched in bioglass particles is also available to some of the inventors of the present invention. The method described in Int J Appl Ceram Technol.,2017,14,1107-1116 concerned the preparation of porous composite scaffolds with controlled pore size from the chitosan/bioglass, sodium alginate/bioglass, polylactide/bioglass system. For the chitosan/bioglass system, the method involved preparing a solution of chitosan in an aqueous acetic acid solution and adding ZnO-subsidised bioglass particles to it. After obtaining a stable dispersion of the bioglass in the chitosan solution, it was lyophilised, after which the resulting porous structure was stabilised using ethanol. The resulting scaffolds were produced as a biomaterial for filling bone defects and tested for biocompatibility and antimicrobial activity.

A chitosan-based material for bone substitutes is described in Polish patent description P411938. A method for obtaining a composite material based on chitosan, hydroxyapatite and silica is disclosed. The method is based on the fact that an aqueous solution of a chitosan salt such as chitosan acetate, chloride, lactate or citrate is mixed with hydroxyapatite nanopowder and possibly calcium glycerophosphate, using ultrasound to obtain a homogeneous paste, which is then introduced into a methsilicic acid sol, the whole is mixed, after which the obtained composite is dried convectively at 50-100 °C.

Patent description US8778378 discloses a material involving bioglass for use as implants capable of preventing surgical site infections, in particular, bioactive antibacterial materials. The disclosure includes bioactive glass with a defined bimodal grain size distribution, flexible and pliable bioactive antibacterial composites made of biocompatible polymer-collagen, ceramics and bioactive glass with a bimodal grain size distribution, as well as bone repair methods and methods to facilitate bone repair while preventing surgical site infection.

Patent description US5977204 discloses biodegradable polymeric materials for therapeutic implants with improved mechanical strength. The described material comprises a surface passivated bioactive glass and a biodegradable polymer selected from a group consisting of polymers: PLA, PGA and copolymers of PLA and PGA. The implants described provide improved mechanical properties and pH control, enabling the use of these materials for the design of porous and non-porous therapeutic implants used as cellular scaffolds for healing tissue defects or fixation devices, with desirable degradation times, mechanical properties, flexibility and biocompatibility.

The use of bioglass for tissue regeneration is disclosed in patent description US11225430. Bioglass fillers in the form of glass beads, glass-ceramic beads and ceramic beads are described, having an internal porous microstructure in the form of a scaffold and surrounded externally by an amorphous coating that allows protection of the porous interior and mechanical reinforcement. The material according to the disclosure is intended to be used in the augmentation or regeneration of bone or soft tissues, where the open porosity present inside the bead will allow an increased degradability in vivo compared to solid particles, will promote tissue growth, including but not limited to all types of bone, soft tissues, blood vessels and nerves.

Bioglass, among others, was also used in the material of patent description US9801946 for synthetic polyurethane composites with osteoconductive properties. According to the description, the composition, in addition to the polyurethane and the osteoconductive component in the form of tricalcium phosphate or bioglass, may contain a growth factor in its composition and may be moldable and/or injectable. After implantation or injection, the composition may be solidified to form a porous composite that provides mechanical strength and promotes cell ingrowth.

Patent description US4895724, on the other hand, discloses a bioglass-free composition for the controlled and prolonged release of macromolecular compounds containing a pure chitosan matrix for injection or in a porous form for implantation, containing a macromolecular compound dispersed therein. Examples of macromolecules used in this composition were pharmacologically active macromolecules selected from the group of proteins and polypeptides, or hormones and enzymes, such as bovine growth hormones, added to the system in an amount of 5-50%. The chitosan in the system was surface crosslinked after obtaining a porous structure by using glutaraldehyde, glyoxal, epichlorohydrin, succinic aldehyde, 1,10-decandial, trichlorotriazine, benzoquinone and bisepoxirane.

A report of the authors of the present invention with regard to a chitosan/bioglass/peptide composite is available in the literature [Int. J. Appl. Ceram. Techn. 2020, 17, 2807-16], but the solution proposed there uses a model peptide with different biological properties than the peptides described in the present description.

Reports of other porous chitosan composites with antimicrobial and/or pro-regenerative and/or anti-inflammatory properties containing bioglass and active biological peptides introduced simultaneously in the bulk and/or on the scaffold surface are unknown.

In addition to bioglass and chitosan, compounds that exhibit pro-regenerative properties by stimulating bone cells to migrate or proliferate are also common components of implants. An extensive literature review on this subject can be found in the authors' publication [Advances in Colloid and Interface Science 276 (2020) 102083].

Patent description US8853165B2 presented a disclosure for a peptide having the ability to both regenerate bone tissue and bind to the surface of the mineral apatite. The peptide was capable of stable immobilisation on the surface of apatite, maintaining effective activity and bone regeneration effect for a long time. Thirty-five peptides that are fragments of growth factors and four peptides with the ability to bind apatite were synthesised. A peptide consisting of an amino acid sequence having the ability to regenerate bone tissue (YGLRSKSKKFRRPDIQYPDAT - growth factor fragment BSP-I) with an amino acid sequence having the ability to bind apatite to itself (STLPIPHEFSRE) was cited as one example of the patent, with the aim of combining the bone-forming effect and the ability to bind to the surface of the mineral.

The invention presented in patent US20170014473A1 describes the therapeutic use of amphiphilic peptides for the treatment and prevention of the progression of osteoporosis and pre-osteoporotic conditions, by direct administration of the peptides to patients with deteriorated or traumatised skeletons, in particular with low bone mineral density. Amphiphilic peptides contain mainly acidic amino acids that are capable, alone or in combination with ions and minerals, of forming β-structure and hydrogels at physiological pH. These peptides serve as scaffolds for mineralisation directly in bone. Compositions containing amphiphilic peptides administered according to the present invention serve as a matrix or nucleation centre for in vitro and in situ biomineralisation. This action is intended to mimic the natural formation of bone tissue, thereby ensuring rapid regeneration, increased mineral density and reduced risk of fractures.

Patent description WO2022084288A1 relates to tissue mineralisation in particular tooth remineralisation and bone regeneration by means of self-organising peptides. The use of the self-organising peptide P11-4 and others in these processes ultimately leads to the formation of hydroxyapatite, which is also found in natural enamel, dentin and bone.

The inventors have discovered therein that both tooth remineralisation and bone regeneration can be significantly accelerated by adding amorphous calcium phosphate or calcium and phosphate ions to the P11-4 peptide, which, when mixed in solution, can lead to the immediate precipitation of calcium phosphate. The disclosure describes a kit comprising a self-organising peptide and calcium and phosphate ions in separate compositions suitable for immediate formation of calcium phosphate precipitates. The disclosure provides a medical application of said kit, in particular in the tooth, for the remineralisation of lesions, mineralisation of pits and fissures.

A combination of pro-regenerative peptides with a polymer is presented in patent description US8546529B2. It relates to an injectable bone regeneration material containing peptides that are fragments of growth factors (BMP-2, BMP-4, BMP-6, BMP-7, BSP-I, BSP-II). The material may comprise one or more peptides from among the 28 shown, and may be bound or mixed with a gel-forming base material selected from the group consisting of: chitosan, alginic acid, silk fibroin, propylene glycol, alginic acid, propylene glycol, poloxamer, chondroitin sulphate and combinations thereof. The injectable bone regeneration material according to the disclosure described in the US can increase the differentiation of bone marrow stromal cells and osteoblasts into bone tissue, thus maximising tissue regeneration. It is in the form of a gel and can therefore be applied to the surface of various medical devices, such as an implant, or mixed with bone graft particles to enhance the treatment effect. The proposed solution also uses peptides derived from other growth factors and presents a different way of combining the peptides with the polymer.

The inconvenience of the known solutions of porous polymer scaffolds enriched in bioglass molecules or the combination of pro-regenerative peptides with polymer/chitosan, is the form that does not provide an environment for cell migration after implantation of the composite, i.e. its effective use for filling bone defects and regenerating bone tissue, as well as the inconvenience of the lack of comprehensive action of such a biocompatible composite - implant. Effective novel biomaterials with a wide range of effects are still in demand and represent an attractive material for medicine.

The invention described in this application presents multifunctional composites containing components with proven pro-regenerative and antimicrobial and/or anti-inflammatory effects simultaneously. The developed composites are biocompatible at the cellular level in vitro and in vivo and are materials that readily adapt to the size and shape of the bone defect.

The invention is disclosed in the claims.

The subject matter of the invention is a biocompatible, non- cytotoxic, bioactive and porous (90% of pores in the range 50-400 μm) composite for filling bone defects and regenerating bone tissue, in which the matrix is a natural biodegradable polymer - chitosan, characterised by a degree of deacetylation DD≥72.6%, viscosity in the range 351 to 2250 mPas and molecular weight in the range 200 to 550 kDa.

Four variants of the composite disclosed herein were developed:

> a) a composite with four components listed: chitosan with bioglass, ug4 peptide and ug46 peptide, in which case it is a four-component composite;

b) composite with three components listed: chitosan with bioglass and ug4 peptide;

c) composite with three components listed: chitosan with bioglass and a peptide designated ug46;

d) composite with three components listed: chitosan with bioglass and peptide fibrils designated UG46 derived from peptide ug46.

Compositions and methods not comprising the peptide sequence referred to as "ug46" or SEQ3 are not part of the invention.

The filler in the composite is a bioactive glass constituting in the composite according to variant a from 8.741 wt.% to 74.106 wt.% of the composition of the total composite, in composite b from 10 wt.% to 75 wt.% of the composition of the total composite, in composite c from 9.92 wt.% to 74.925 wt.% of the composition of the total composite, and in composite d from 9.95 wt.% to 74.925 wt.% of the composition of the total composite, produced by the sol-gel method and containing in total: $SiO_2$ in an amount of 60-75 wt.% and CaO in an amount of 10-30 wt.% and, in a variant disclosed herein, additionally: $P_2O_5$ in an amount of up to 8 wt.% and/or SrO in an amount of up to 7 wt.% and/or ZnO in an amount of up to 7 wt.% and/or CuO in an amount of up to 3 wt.% and/or MgO in an amount of up to 3 wt.%. The bioglass has a grain size of 90% below 250 μm and is characterised, due to the choice of composition, by enhanced bioactivity, bactericidal activity and/or pro-regenerative capacity.

The entire system, i.e. the composite in variant a, is enriched with two biologically active, engineered peptides - ug4 and ug46 with specific amino acid sequences, shown on SEQ1 - with pro-regenerative properties and SEQ3 - with antimicrobial and anti-inflammatory properties.

The ug4 peptide is between 1.130 wt.% and 11.5596 wt.% of the total composition of the composite a. A peculiarity of the design of the engineered ug4 peptide is that a portion of this peptide - that is, a peptide called peptide ug1 with the sequence shown in SEQ2 - will be released at the site of medical action by enzymatic hydrolysis of the bond of the peptide in ug4 between $Gly^4$ and $Leu^5$. The enzymatic cut between these amino acid residues is specific for metalloproteinases found in the bone environment. The ug1 peptide as part of ug4 has pro-regenerative activity in bone.

The peptide ug46 is between 0.044 wt.% and 1.575 wt.% of the total composition of the composite and exhibits antimicrobial and anti-inflammatory properties.

The entire system, i.e. the composite in variant b, is enriched with one biologically active engineered peptide - ug4 with a specific amino acid sequence, shown on SEQ1 - with pro- regenerative properties. The ug4 peptide is between 1.129 w.t% and 13.64 wt.% of the total composition of the composite in composition b.

The entire system, i.e. the composite in variant c, is enriched with a biologically active, engineered peptide, ug46, with the specific amino acid sequence shown on SEQ3, with antimicrobial and anti-inflammatory properties introduced in an amount of 0.1 wt.% to 0.8 wt.% of the total composition of the composite.

The entire system, i.e. the composite in variant d, is enriched with a biologically active, engineered peptide with the specific amino acid sequence shown on SEQ3, in the form of peptide fibrils - UG46, obtained by a known thermal incubation method, with antimicrobial properties, introduced in an amount of 0.1 wt.% to 0.5 wt.% relative to the total composition of the composite.

As for the amount of polymer used in the composite - chitosan, in each of the composite variants described - a, b, c, d it represents:

in composite a (chitosan/bioglass/peptide ug4/peptide ug46) from 24.038 wt.% to 86.256 wt.% of the total composite composition,

in composite b (chitosan/bioglass/ ug4 peptide) from 23.871 wt.% to 76.36 wt.% of the total composition of the ternary composite,

in composite c (chitosan/bioglass/peptide ug46) from 24.80 wt.% to 89.91 wt.% of the total composite composition,

in composite d (chitosan/bioglass/peptide fibrils UG46) from 24.88 wt.% to 89.91 wt.% of the total composite composition.

The essence of the solution according to the disclosure is also a method of obtaining this composite for filling bone defects and regenerating bone tissue, in which the matrix is a natural biodegradable polymer - chitosan, characterised by a deacetylation degree of DD≥72.6%, a viscosity in the range of 351 to 2250 mPas and a molecular weight in the range of 200 to 550 kDa, containing bioglass and two engineered peptides: ug4 and ug46 or one engineered peptide: ug4 or one engineered peptide: ug46 or peptide fibrils: UG46. The bioactive component in the form of a bioglass with antimicrobial and/or pro- regenerative properties is introduced into the composite at the further described step during the production of the stable dispersion after it has been obtained in an amount ranging from 8.741 wt.% to 74.106 wt.%. of the total composition of the composite - for variant a, in an amount from 10 wt.% to 75 wt.% of the total composition of the composite - for variant b, in an amount from 9.92 wt.% to 74.925 wt.% of the total composition of the composite in variant c and in an amount from 9.95 wt.% to 74.925 wt.% of the total composition of the composite in variant d.

The biologically active component in the form of the pro-regenerative peptide ug4 in composite variants a and b is introduced into the composite at the step described below in an amount ranging from 1.130 wt.% to 11.559 wt.% of the total composition of the composite for variant a and from 1.129 wt.% to 13.64 wt.% of the total composition of the composite for variant b, in the form of chitosan modified with this peptide by covalent bonding to this polymer.

The biologically active ingredient - the antimicrobial and anti-inflammatory peptide ug46 in composite variants a and c - is introduced into the composite at the stage described below by surface adsorption: for variant a on the porous chitosan/bioglass/ug4 peptide scaffold in an amount of 0.044 wt.% to 1.575 wt.% relative to the total composition of the composite, for variant c on the porous chitosan/bioglass scaffold in an amount of 0.1 wt.% to 0.8 wt.% relative to the total composition of the composite, or in a variant of composite d is introduced during the manufacture of the stable dispersion in the form of previously obtained peptide fibrils with antimicrobial activity of UG46 by the known method described previously DOI:10.3390/ijms22083818 in an amount of 0.1 wt.% to 0.5 wt.% relative to the total composition of the composite.

The method of obtaining the composite varies depending on the composition of the composite and how the biologically active peptide is introduced.

I. In the case of the four-component composite (chitosan/bioglass/peptide ug4/peptide ug46) - variant a composition, a first step prepares by sol-gel method a bioglass whose composition ranges from $SiO_2$ in an amount of 60-75 wt.% and CaO in an amount of 10-30 wt.%, and: $P_2O_5$ in an amount of 0-8 wt.%, and/or SrO in an amount of 0-7 wt.%, and/or ZnO in an amount of 0-7 wt.%, and/or CuO in an amount of 0-3 wt.%, and/or MgO in an amount of 0-3 wt.%. After mixing the bioglass synthesis precursors, the reaction mixture is subjected to slow drying in a drying oven gradually increasing the temperature from 40 °C to 180 °C. The dried gel is then heated in an electric oven at temperatures from 500 °C to 750 °C and subjected to grinding to a grain size where 90% of the grains of the resulting bioglass are below 250 $\mu$m.

In the second step, the ug4 peptide with the sequence Cys Pro Leu Gly Leu Tyr Gly Phe Gly Gly is produced by synthesis on a solid support, e.g. SPPS using the Fmoc methodology, using an automated microwave synthesiser, e.g. Liberty Blue from CEM Corporation. The reaction to cleave the peptide from the carrier with simultaneous removal of the side chain sheaths is carried out using a reaction mixture consisting of trifluoroacetic acid, phenol, water, 1,2-ethanedithiol and triisopropylsilane. The peptide is subjected to purification using reversed-phase high-performance liquid chromatography.

In the third step - for composite a - the ug4 peptide is attached to the chitosan by the well-known click chemistry method at a rate of 3.2 g per 3 g of chitosan. This method consists in attaching ug4 peptide with the sequence Cys Pro Leu Gly Leu Tyr Gly Phe Gly Gly to the chitosan modified with maleimidoglycine. Modification of chitosan by maleimidoglycine follows the following procedure. Chitosan, e.g. 95/2000 [1000 mg, 0.61 mmol, 163.1 g/mol], was suspended in 700 to 800 ml of hot water and then stirred using e.g. a magnetic stirrer until the solution reached room temperature. The N-hydroxysuccinimidyl ester of aminoacetic acid, e.g. 154.34 mg, 0.3 mmol, was dissolved in tetrahydrofuran 110 to 120 ml relative to the weighed ester for 10 to 15 min under ultrasound, then 80 to 90 ml of water was added, e.g. final volume water + THF=200 ml. After at least 24 h, the modified chitosan was drained. The modified chitosan was then washed with very cold water depending on the amount of chitosan, e.g. 1 l of water per 3.2 g of chitosan, and lyophilised with acetic acid.

Chitosan modified with peptide ug4 is produced by modification using a covalent bond between the peptide and a maleimide linker attached to the chitosan. Chitosan with a degree of maleimidoglycine modification of 9% to 10% is suspended in water and a concentrated peptide solution is added. The mixture is brought to a pH of 8.2 to 8.5 with sodium bicarbonate and maintained in an argon-filled environment at room temperature with continuous stirring for at least 24 hours. The chitosan is then drained The resulting product is washed with very cold water at a rate of 1 l of water per 1 g of modified chitosan. The peptide-modified chitosan is lyophilised.

The assumption for chitosan modified with ug4 peptide is that part of this peptide - that is, a peptide called ug1 peptide - will be released from chitosan by enzymatic hydrolysis of the peptide bond in ug4 between Gly[4] and Leu[5]. The enzymatic cut between these amino acid residues is specific for metalloproteinases found in the bone environment. In order to test the biological activity of the peptide released from chitosan, a peptide with the sequence Leu Tyr Gly Phe Gly Gly (ug1) was synthesised and for which cytotoxicity and pro-regenerative activity tests were performed.

In the fourth step for variant a, a solution of the mixture of chitosan and chitosan modified with ug4 peptide - obtained in the third step - is prepared at a concentration of 0.5-4 wt.%, in an acidic aqueous solution, in which the weight ratio of chitosan to chitosan modified with peptide ug4 is from 1.130 to 11.299. At the same time, a paste is prepared on the basis of bioglass particles of known composition and grain size wetted with an acidic aqueous solution with a particle concentration in the range of 0.02 to 0.42 g per 1 ml of solution, after which the produced paste is introduced into the chitosan solution and mixed e.g. magnetically for 5-60 minutes at room temperature with the formation of a stable dispersion of the bioglass particles in the acidic chitosan solution with the peptide in this solution so prepared. The produced suspension is then transferred to a mould of any shape and size and subjected to a freeze-drying process to obtain a porous composite structure, the whole system being stabilised by chemical or physical crosslinking before or after the freeze-drying process, and the finished porous structures being washed to neutral pH and freeze-dried again.

In the fifth step for variant a, a peptide ug46 with the sequence Ac Gln Ala Gly Ile Val Val Pro Leu Gly Leu Gly Leu Leu Lys Arg Ile Lys Thr Leu Leu is produced by synthesis on a solid support, e.g. SPPS using the Fmoc methodology, using an automated microwave synthesiser, e.g. Liberty Blue from CEM Corporation. The reaction to cleave the peptides from the carrier with simultaneous removal of the side chain sheaths is carried out using a reaction mixture consisting of trifluoroacetic acid, phenol, water, 1,2-ethanedithiol and triisopropylsilane. The peptide undergoes purification using reversed-phase high- performance liquid chromatography.

In the sixth step, an aqueous solution of peptide ug46 at a concentration of 0.25 to 0.35 mg/ml is prepared and applied by adsorption to the surface of the porous chitosan/bioglass/peptide ug4 structures by immersing the porous scaffold in the peptide ug46 solution for 4-24 hours and again drying the porous composite by freeze-drying.

II. For composition variant b (chitosan/bioglass/ug4 peptide), the process follows the methodology described above - from step one to step four in the same way as for variant a.

III. In the case of the composition variant c (chitosan/bioglass/peptide ug46), a bioglass is produced in the first step for variant c - description of the methodology as described above - first step for variant a, and then the second step is carried out, i.e.: a solution of chitosan of 0.5-4 wt.% is prepared in an acidic aqueous solution and at the same time a paste based on bioglass particles of known composition and grain size, wetted with an acidic aqueous solution, with a particle concentration in the range of 0.02 to 0.42 g per 1 ml of solution is prepared, after which the paste produced is introduced into the chitosan solution and mixed e.g. magnetically for 5-60 minutes at room temperature to produce a stable dispersion of the bioglass particles in the acidic chitosan solution, and then the suspension is transferred into a mould of any shape and size and lyophilised to obtain a porous composite structure, the entire system is subjected to stabilisation by chemical or physical crosslinking before or after the lyophilisation process, and the finished porous structures are subjected to washing to an inert pH and freeze-drying again. In the third step, the ug46 peptide is produced - description of the methodology identical to that described above - step five for variant a, and then a fourth step analogous to step six for variant a is carried out.

IV. For the composition variant d (chitosan/bioglass/UG46 peptide fibrils), a bioglass is prepared in the first step - description of the methodology as described above - step one for variant a. In the second step, the ug46 peptide is produced with the sequence Ac Gln Ala Gly Ile Val Val Pro Leu Gly Leu Leu Leu Lys Arg Ile Lys Thr Leu Leu, as described in step five for variant a. In step three, UG46 peptide fibrils are produced using the thermal incubation method by dissolving the ug46 peptide in PBS phosphate buffer and placing the sample in a thermal block and incubating for 7 days at 37 °C using continuous shaking (300 rpm), as described in publication : DOI: 10.3390/ijms22083818. The fourth step is then carried out analogously to the description for step two for variant c, except that after a stable dispersion of the bioglass in the acidic chitosan solution has been formed, a solution of UG46 peptide fibrils is introduced into the system and the whole is further mixed to homogenisation, and the process is then further carried out as in the description of the methodology as for step two for variant c.

Advantageously for the method of producing all four composite variants (a, b, c, d), the acidic aqueous solution for dissolving the chitosan and producing a paste based on the bioglass particles is a 0.5-5% aqueous solution of acetic or lactic or malic or oxalic acid.

Advantageously for compositions a and b, the occupancy of the chitosan chains with the ug4 peptide is 0.8-3.4%.

Advantageously for compositions a and c, the percentage of active peptide ug46 introduced on porous structures by surface adsorption is 0.044- 1.575%.

Advantageously for the manufacturing method in variants a and c, the concentration of the solution of peptide ug46 introduced on the porous structures by surface adsorption is between 0.25 and 0.35 mg/ml.

Advantageously for the manufacturing method in variant d, the concentration of the aqueous solution of peptide fibrils UG46 introduced into the system is between 0.80 and 0.85 mg/ml.

Advantageously for the method of manufacturing all four composite variants (a, b, c, d), in order to obtain a stable porous structure, the crosslinking of the chitosan chains is carried out during the manufacture of the composite matrix prior to the lyophilisation process using a 5% ethanolic solution of genipin added to the composite mass in a chitosan/genipin weight ratio ranging from 1:0.01 to 1:0.043, mixing the composite matrix with the genipin solution and conditioning the system for 1-24 hours at 30-80 °C or using a 37.5% solution of 5-water disodium β-glycerophosphate (BGP) added to the composite mass placed in an ice bath, in a weight ratio of chitosan/BGP ranging from 1:2 to 1:5, mixing the composite matrix with the BGP solution and conditioning the system for 1-24 hours at 30-80 °C or in order to obtain a stable porous structure, freeze-dried porous structures not crosslinked during the manufacture of the composite matrix shall be stabilised after the freeze-drying process by immersion in 70-99.8% ethanol for 1-24 hours or by crosslinking with the an aqueous solution of 5-water sodium tripolyphosphate with a concentration in the range 0.1- 0.4 M or an ethanolic solution of vanillin with a concentration in the range 1- 5%, by immersing the scaffolds in these solutions for 1-24 hours or by the dehydrothermal method by conditioning the porous scaffolds under reduced pressure at a temperature in the range 40-105 °C for 24-96 hours.

Advantageously for the manufacturing method, the finished stable porous structures of the composite are washed for 10-60 min using 0.1 M NaOH and 6-24 hours to a pH in the range of 6.5 - 7.5 using deionised water or 6-24 hours to a pH in the range of 6.5 - 7.5 using deionised water.

In order to make use of the invention and to obtain a usable form of the porous composite (individual, personalised shape and dimension, adapted to the shape and dimension of the bone defect), a suspension is poured into a mould of the desired shape and dimension and then freeze-dried, or by mechanical processing (e.g. cutting or shearing) of the finished porous composite obtained by freeze-drying.

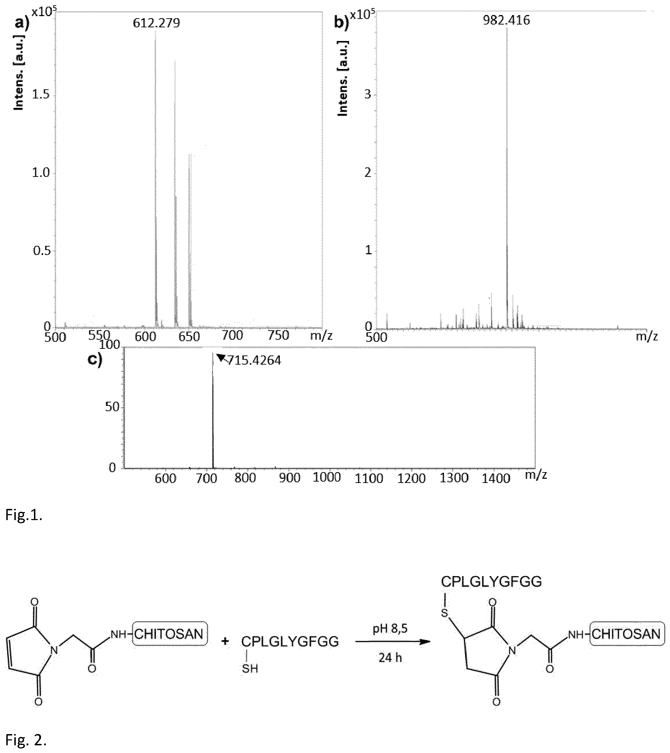

The object of the invention is illustrated in examples 1-20 and in figures 1-22, wherein compositions and methods not comprising the peptide sequence referred to as "ug46" or SEQ3 are not part of the invention; in particular

Fig. 1. shows the MALDI-TOF mass spectra of the peptides: a) ug1 - 612.279 [M+H], 634.272; [M+Na], 650.251 [M+K]; b) ug4 - 982.416, c) ug46 - mass after deconvolution (2142.2602) - multiply charged ion: 715.0924 $[M+3H]^{3+}$, shows the reaction scheme for the attachment of ug4 peptide to modified chitosan

Fig. 2. shows the reaction scheme for the attachment of ug4 peptide to modified chitosan,

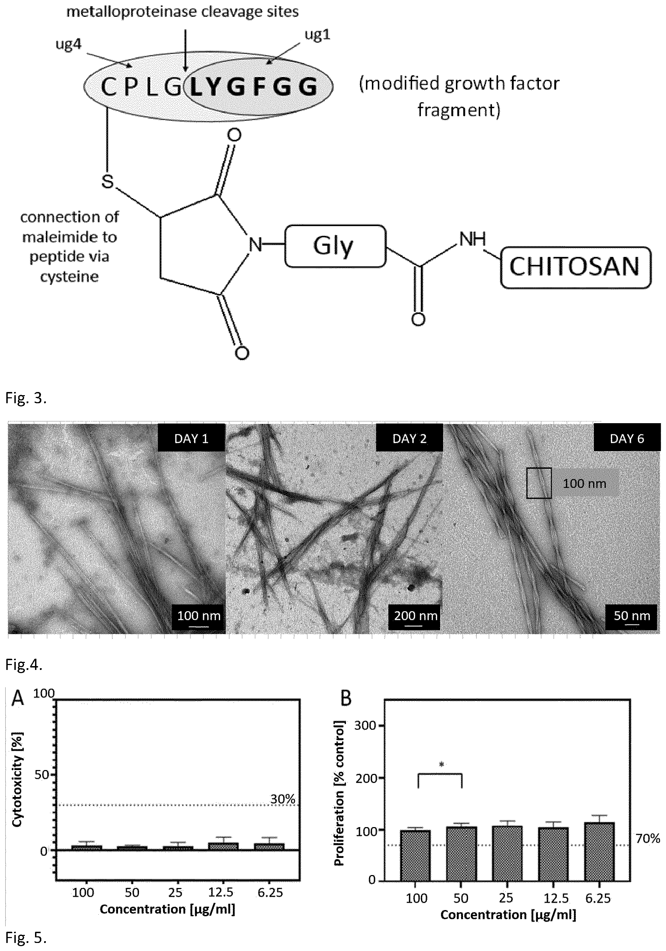

Fig. 3. shows a schematic of the modified chitosan, where the attached ug4 peptide consists of two fragments - a metalloproteinase VII-specific sequence and the active sequence (ug1 peptide),

Fig. 4. shows TEM (transmission electron microscopy) images of UG46 peptide fibrils formed during a 7-day thermal incubation,

Fig. 5. shows: a) cytotoxicity towards hFOB 1.19 cells induced by the presence of ug1/ug4 peptides of different concentrations (asterisks indicate statistically significantly different ($p < 0.05$)), b) proliferation of hFOB 1.19 cells in the presence of ug1/ug4 peptides at different concentrations (asterisks indicate peptide concentrations that induced proliferation with values that were statistically significant differences ($p < 0.05$)),

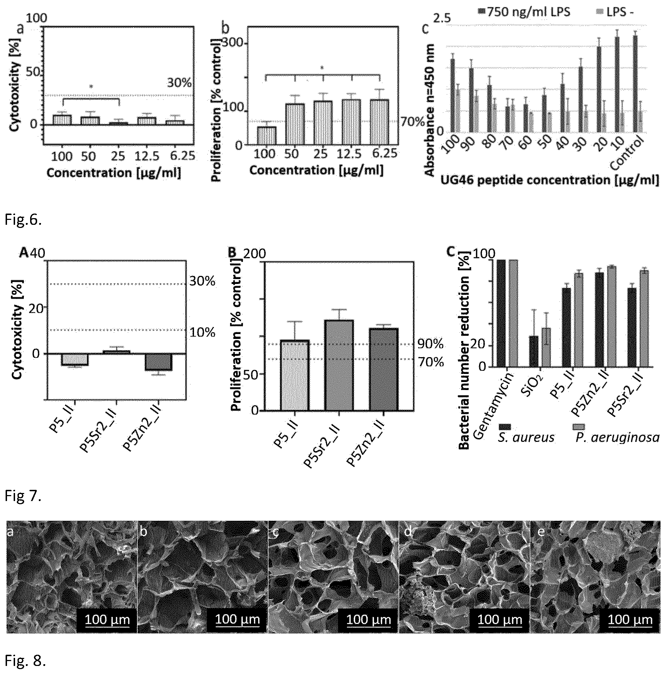

Fig. 6. shows: a) cytotoxicity of hFOB 1.19 cells induced by the presence of peptide ug46 at different concentrations. Asterisks indicate statistically significant differences ($p < 0.05$). b) proliferation of hFOB 1.19 cells in the presence of different concentrations of peptide ug46 . Asterisks indicate statistically significant differences ($p < 0.05$), (c) Graph: amount of interleukin 8 in medium from WI-38 cells incubated in the presence of different concentrations of peptide ug46 in medium with and without LPS

Fig. 7. shows: A- cytotoxic effect of glasses in contact with hFOB cells 1.19 (ATCC CRL-11372, USA); B- proliferation of hFOB 1.19 cells in contact with glasses as determined by the WST-1 test (Abcam, USA); C- bactericidal effect of glasses in contact with S. aureus and P. aeruginosa expressed as a reduction [%] in the initial bacterial count, gentamicin and silicon oxide were used as a control,

Fig. 8. shows the microstructure of chitosan-bioglass composites with ug4 peptide covalently attached to chitosan: a - (CH_CH_ug4_0.8_P5Zn2II); b - (CH_CH_ug4_1.6_P5Zn2ll); c - (CH_CH_ug4_3.4_P5Zn2ll); d - (CH_CH_ug4_3.4_P5Sr2II); e - (CH_CH_ug4_3.4_P5Zn2II_ug46),

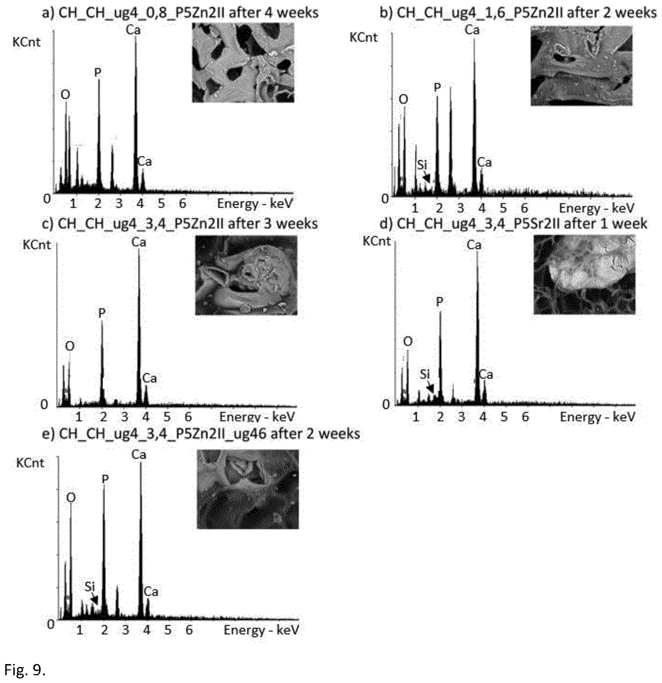

Fig. 9. shows EDS spectra and SEM images of bioglass areas in chitosan-bioglass composites with the addition of ug4 peptide covalently attached to chitosan: a - (CH_CH_ug4_0.8_P5Zn2ll); b - (CH_CH_ug4_1.6_P5Zn2II); c - (CH_CH_ug4_3.4_P5Zn2ll); d - (CH_CH_ug4_3,4_P5Sr2II); e - (CH_CH_ug4_3,4_P5Zn2ll_ug46) incubated in SBF,

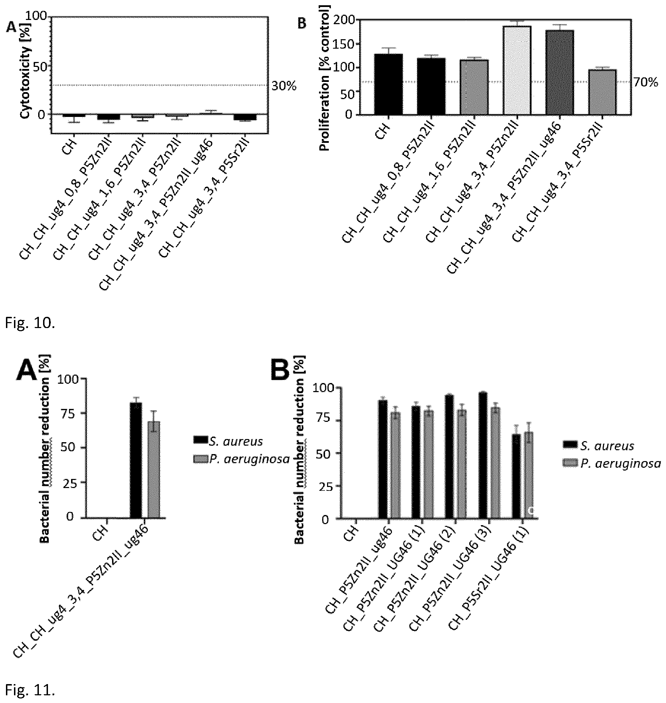

Fig. 10. shows: A- cytotoxic effect of chitosan-bioglass composites with ug4 peptide covalently attached to chitosan in contact with hFOB 1.19 cells (ATCC CRL-11372, USA). B- proliferation of hFOB 1.19 cells in contact with chitosan-bioglass composites containing ug4 peptide covalently attached to chitosan as determined by the WST-1 assay (Abcam, USA),

Fig. 11. shows the reduction values [%] in the number of bacteria determined for the chitosan-bioglass composites: with the addition of ug4 and ug46 peptides, with the addition of ug46 peptide, with the addition of UG46 peptide fibrils, against the bacteria S. aureus PCM 2602 and P. aeruginosa PCM 2563.

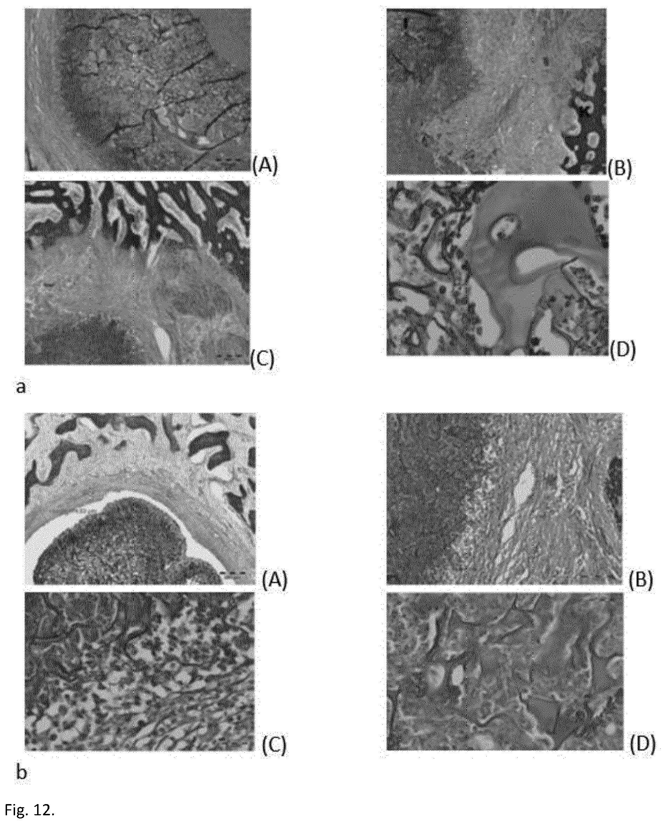

Fig. 12. shows a microscopic image 30 days after implantation: a - of the composite CH_CH_ug4_3.4_P5Zn2II_ug46: (A) brown-coloured implant visible (Pow. 40x.Colours.VG.), (B) upper left - implant separated from the cancellous bone tissue by a connective tissue band (Pow. 40x.VG stain), (C) central - band of connective tissue separating the brown-coloured implant from the cancellous bone tissue (Pow. 40x.VG stain), (D) foci of ossification within the implant (Pow.400HE stain); b - composite CH_P5Zn2II_UG-46 (1): (A) bottom left - visible brown stained implant separated from red staining bone tissue by connective tissue capsule (Pow.40x.HG stain), (B) inflammatory granulation tissue with exudate-edge discontinuities within the implant (Pow. 400x.Colour.VG), (C, D) centre of the implant with foci of mineralisation (Pow. 400x.Colour.VG),

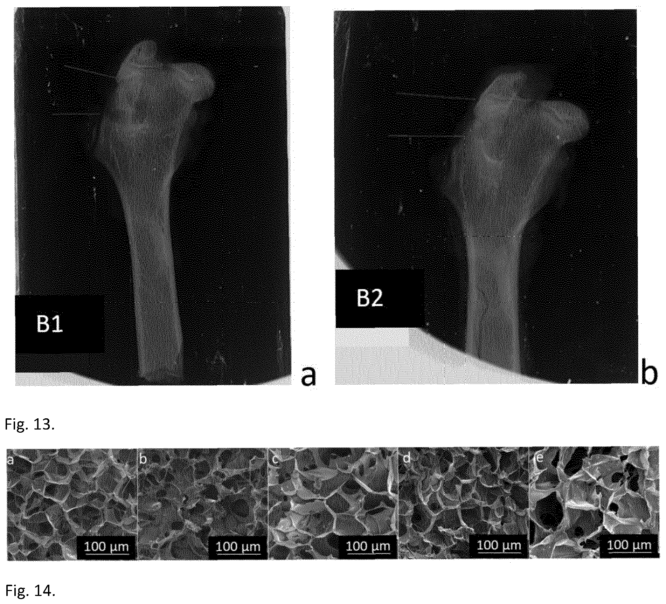

Fig. 13. shows a radiological image of the site, 30 days after implantation: a - composite CH_CH_ug4_3.4_P5Zn2II_ug46, b -composite CH_P5Zn2ll_UG- 46 (1),

Fig. 14. shows the microstructure of the chitosan-bioglass composites with the addition of the peptide in the form of UG46 fibrils: (a - (CH_P5Zn2ll_UG-46 (1)); (b - (CH_P5Zn2II_UG-46 (2)); (c - (CH_P5Zn2II_UG-46 (3)); (d - (CH_P5Sr2II_UG-46 (1)); (e - microstructure of the chitosan-bioglass composite with peptide ug46 introduced into the system by surface adsorption (CH_P5Zn2II_ug46),

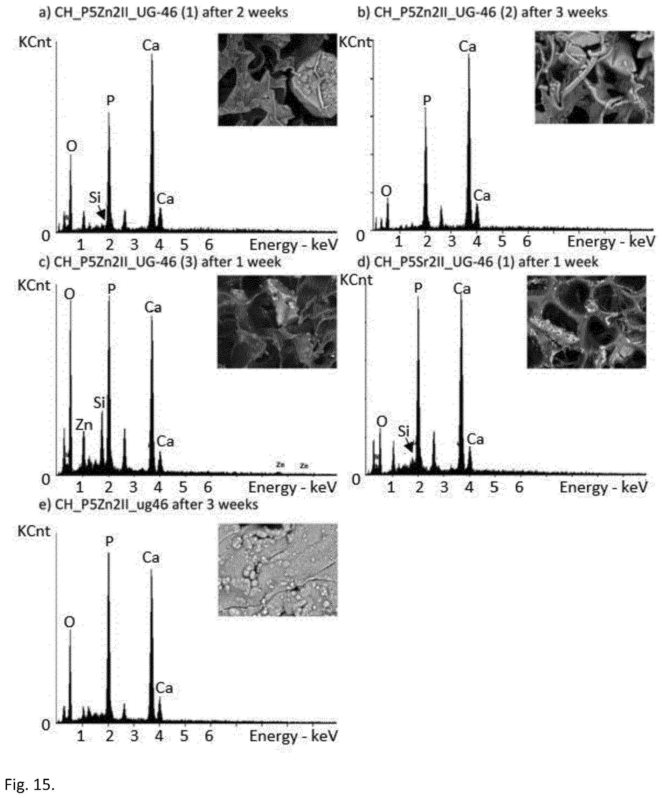

Fig. 15. shows EDS spectra and SEM images of bioglass areas in chitosan-bioglass composites with peptide in the form of UG46 fibrils: a -(CH_P5Zn2ll_UG-46 (1)); b - (CH_P5Zn2II_UG-46 (2)); c - (CH_P5Zn2II_UG-46 (3)); d - (CH_P5Sr2II_UG-46 (1)); e - EDS spectrum and SEM image of bioglass regions in a chitosan-bioglass composite with ug46 peptide introduced into the system by surface adsorption (CH_P5Zn2II_ug46), incubated in SBF;

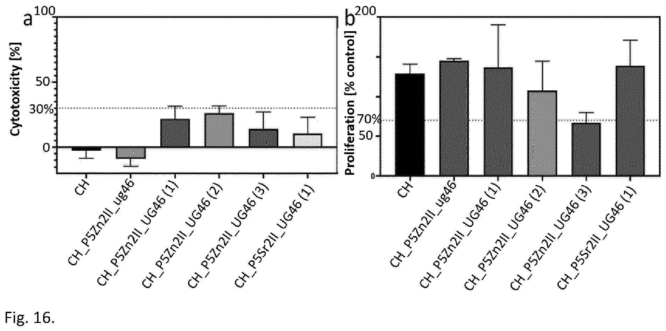

Fig. 16. shows: A- Cytotoxic effect of chitosan-bioglass composites with UG46 fibril peptide and with ug46 peptide introduced into the system by surface adsorption in contact with hFOB 1.19 cells (ATCC CRL-11372, USA). B- Proliferation of hFOB 1.19 cells in contact with chitosan-bioglass composites with UG46 fibril peptide and with ug46 peptide introduced into the system by surface adsorption as determined by the WST-1 assay (Abcam, USA).

Example 1. SYNTHESIS OF PEPTIDES, DISCOVERY OF PEPTIDES FROM THE CARRIER AND PURIFICATION AND REPLACEMENT OF THE COUNTERION

Peptides were synthesised on solid support (SPPS) using Fmoc methodology, using CEM Corporation's Liberty Blue automated microwave synthesiser with a constant flow of reagents on Rink Amide ProTide Resin with a capacity 0.59 mmol/g (1.69 g, 1.0 mmol). The peptides were cleaved from the resin with simultaneous removal of side chain sheaths was carried out using a trifluoroacetic acid (TFA), phenol, water, 1,2-ethanedithiol (EDT) and triisopropylsilane (TIPSI) (91.5:5:5:2.5:1; v/v/v/v/v/v). For 1 g of resin, 10 ml of the mixture was used. The reaction was carried out for three hours using a laboratory shaker, and then the resin was drained. The filtrate was concentrated using a vacuum evaporator. The crude product was precipitated from the mixture using diethyl ether. The resulting precipitate was centrifuged for 20 minutes at 4 °C at 4000 rpm. The crude and dry product was dissolved in water and subjected to sublimation drying using a freeze-dryer.

Peptides were purified using reversed-phase high-performance liquid chromatography (chromatograph consisting of two pumps (K1001) and a UV detector (K-2001) coupled to a Gilson collector) using a semipreparative Jupiter® Proteo-90-4-C8 column (Phenomenex) (21.2 mm × 250 mm). Chromatographic separation was carried out in a linear gradient. The mobile phase was a solvent system:

- A - 0,1 M $CH_3COOH$, 0,1 M $CH_3COONH_4$ in $H_2O$
- B- 65% $CH_3CN$ in 0,1 M $CH_3COOH$, 0,1 M $CH_3COONH_4$ in $H_2O$ (v/v)

The eluent flow rate was 15 ml/min, UV detection at $\lambda$ = 223 nm.

Example 2. CHARACTERISTICS OF THE PEPTIDES RECEIVED

Reversed-phase high-performance liquid chromatography (Nexera X2 chromatograph, Shimadzu) was used to determine the purity of the synthesised peptides using an analytical Kromasil-300-5-C8 column (4.6 × 250 mm), volumetric flow rate was 0.5 ml/min, eluents:

- A - 0,1% TFA in $H_2O$ (v/v)
- B- 80% $CH_3CN$ in 0,1% TFA in $H_2O$ (v/v).

The identity of peptides was confirmed by Bruker Briflex III MALDI-TOF spectrometer from Bruker Daltonics and/or LCMS-ESI-IT-TOF on a Kromasil-100-5- C8 column (1 mm × 250 mm). The theoretical monoisotopic molecular weight of the compounds, experimental weights and retention times are shown in Table 1. The mass spectra of the purified peptides are shown in Fig. 1.

Table 1. Theoretical monoisotopic compound molecular weight, experimental weight and retention times.

| name | sequence | retention time | Theoretical mass | Experimental mass |
|---|---|---|---|---|
| ug1 | Leu Tyr Gly Phe Gly Gly | 4.62 | 611.6986 | 611.279 |
| ug4 | Cys Pro Leu Gly Leu Tyr Gly Phe Gly Gly | 5.92 | 982.1716 | 981.428 |
| ug46 | Ac Gln Ala Gly Ile Val Val Pro Leu Gly Leu Gly Leu Leu Lys Arg Ile Lys Thr Leu Leu | 10.12 | 2142.3979 | 2142.2590 |

Example 3. PROCEDURE FOR CONNECTING MALEIMIDOGLYCIN TO CHITOSAN AND CONNECTING MALEIMIDOGLYCIN-MODIFIED CHITOSAN PEPTIDE AND QUANTITATIVE ANALYSIS OF CONNECTED PEPTIDE

Chitosan 95/2000 [1000 mg, 0.61 mmol, 163.1 g/mol] was suspended in 800 ml hot water - stirred with a magnetic stirrer until the solution reached room temperature.

Aminoacetic acid N-hydroxysuccinimide ester [154.34 mg, 0.3 mmol] was dissolved in 120 ml of tetrahydrofuran (15 min on ultrasound), then 80 ml of water was added. The solution thus prepared was added to the chitosan suspension. The whole mixture was left for 20 min with intensive stirring using a magnetic stirrer. After this time, the pH was brought to a value of 8.3 with sodium hydrogencarbonate. The mixture was left for 24 h with continuous stirring. After 24h, the modified chitosan was drained on a Schott funnel with the smallest possible pores. The modified chitosan was then washed with very cold water about 1000 ml for this amount of chitosan. The washed chitosan was lyophilised from acetic acid. The ug4 peptide is then attached to the chitosan by a known click chemistry method at a rate of 3.2 g per 3 g of chitosan. This method involves the attachment of ug4 peptide with the sequence Cys Pro Leu Gly Leu Tyr Gly Phe Gly Gly to maleimidoglycine - modified chitosan. Chitosan with a 10% degree of maleimidoglycine modification (3.2 g) was placed in a round-bottomed flask and 500 ml of water was added. A solution of ug4 peptide (3 g ug4 peptide was dissolved in deionised water) was added to the previously prepared suspension. The mixture was brought to pH~8.5 by adding 25 ml of NaHCO$_3$. Once the correct pH was achieved, a balloon filled with argon was placed over the neck of the flask to limit the oxidation of the free cysteine located at the N-terminus of the attached peptide. The reaction mixture was left at room temperature with continuous stirring for 24 h (Fig. 2). The chitosan was then drained using an oozing kit under reduce. The resulting product was washed with very cold water (1 l of water per 1 g of modified chitosan). The peptide-modified chitosan was lyophilised.

The modified chitosan was analyzed for the degree of deposition. The procedure for the determination of the peptide differs from that used in the above-mentioned patent application. In this procedure the addition of the internal standard solution and the amino acid to be determined, i.e. a 0.1 M solution of lysine in 6 M hydrochloric acid was used to quantify the peptide attached to the chitosan. The amount of internal standard, was recalculated so that the mass of lysine added was equal to the expected mass of one of the amino acids contained in the attached peptide (leucine) contained in the sample. Samples after the hydrolysis procedure were analysed by high-performance liquid chromatography on normal phases using a Shimadzu NEXERA X2 chromatograph using a Phenomenex BioZen column of size: 4.6 × 100 mm. The flow rate was 0.25 ml/min. The composition of the mobile phases was: System A: 0.5 M ammonium formate, 0.5 M formic acid, 90% ACN in water and System B: 0.5 M ammonium formate, 0.5 M formic acid, 75% ACN in water. The time for a single analysis was 45 min. To determine the degree of deposition, the ratio of leucine to lysine in the sample should be compared to the ratio of these amino acids from a standard curve of their mixture of equal and known masses.

Three samples (10 mg) of modified chitosan were subjected to the hydrolysis reaction, and three chromatographic analyses were performed for each sample. The ratio of the area under the leucine to lysine peak on the common chromatogram was compared with the determined ratio of these amino acids from a standard curve performed under the same conditions as the analysis of the target samples. The results are shown in Table 2. From the data obtained, it can be seen that maleimidoglycine bound to chitosan was fully deposited with peptide ug4 (Fig. 3).

Table 2. Results of chromatographic analysis of chitosan samples modified with ug4 peptide (determined from the standard curve, Leu/Lys ratio was 0.813985).

| Sample | Ratio of area under peak Leu to Lys | Average degree of chitosan peptide deposition |
|---|---|---|
| CH-ug4-I | 0.818345 | 100.54% |
| CH-ug4-II | 0.720802 | 88.55% |
| CH-ug4-III | 0.909041 | 111.68% |
| | | 100.26% |

Example 4. THE PROCEDURE FOR PRODUCING PEPTIDE FIBRILS AND THEIR MORPHOLOGICAL CHARACTERISTICS

The ug46 peptide was thermally incubated to obtain mature peptide fibrils. For this purpose, 1 mg of the peptide was dissolved in 1 ml of PBS phosphate buffer. The sample was placed in a thermal block and incubated for 7 days at 37 °C using continuous shaking (300 rpm). On subsequent days of incubation, samples were taken for characterisation of the growing peptide fibrils, starting when the peptide was dissolved in PBS buffer. The collected samples were analysed using the following techniques: atomic force microscopy (AFM), transmission electron microscopy (TEM) and thioflavin assay.

Microscopic analysis (TEM) of samples taken on consecutive days of incubation confirmed that the process of peptide fibre formation begins immediately after the peptides are dissolved in PBS buffer. The analysis was performed by placing a small amount of peptide on a microcellulose mesh and then staining with 2% uranyl acetate. Images were taken using a TECNAI SPIRIT BIO TWIN FEI microscope at 120 kV excitation. The images show that already after 24 hours of incubation, the formed ug46 peptide fibers reach a length of 100 nm to 1000 nm. During the following days of incubation, the formed protofibrils become organised, as evidenced by the mature filaments visible on day 6 of incubation, consisting of several parallel filaments that twist around their axis. The twisting of the fibrils visible in the images is reproducible and occurs throughout the length of the formed fibrils every 100 nm (Fig.4).

In the next experiment, the samples taken during incubation with the fluorescent dye thioflavin T were measured. The analysis was carried out to confirm the presence of $\beta$-structures, which form a complex with thioflavin T to give a characteristic emission spectrum at 482 nm. The spectra were recorded on a Tekan Infinite 200Pro spectrofluorimeter in COSTAR Flat plate Black 96-well plates. A peptide solution of 0.1 mg/ml in PBS with thioflavin T solution, whose final concentration in the sample was 10 mM, was used for the measurement. The negative control was a PBS solution with thioflavin T. The sample was excited at 420 nm and the emission spectrum was recorded between 455 and 600 nm. The absorption maximum was at 482 nm. The increase in fluorescence emission intensity of the complex T peptide-thioflavin with advancing incubation time reaches its maximum at day 6 of incubation.

In addition, an experiment was performed to incubate previously obtained peptide fibrils in the presence of the enzyme metalloproteinase VII. The enzyme MMP-7 (enzyme:substrate molar ratio used - 1:365.6) (Sigma Aldrich, St. Louis, MO, USA) was added to ug46 fibrils formed by aggregation at a concentration of 1 mg/ml in PBS. The incubation process was carried out for 72 h at 37 °C with agitation. Samples were taken at the following time points 0, 1, 3, 24, 72 h, and a 10% TFA solution was added to each sample to inhibit the digestion reaction. Experimental results were analysed using a Bruker Briflex III MALDI-TOF spectrometer from Bruker Daltonics.

MS analysis showed that, under the influence of the enzyme, a peptide containing the active sequence was released from the fibril. Enzymatic cutting occurred at the expected cutting site, i.e. between Gly and Leu residues, in the Pro Leu Gly Leu sequence specific for MMP-7. Two main products of enzymatic hydrolysis were observed in the MALDI mass spectra: an active sequence with an enzyme-specific fragment (Leu Gly Leu Leu Lys Arg Ile Lys Thr Leu Leu; 1225.759 [M+ H]) and a second fragment (Ac Gln Ala Gly Ile Val Val Pro Leu Gly; 917.5 [M+Na]$^+$ ) comprising a fibrillogen sequence (Ac Gln Ala Gly Ile Val Val) and a part of the peptide with an MMP-7-specific fragment (Pro Leu Gly).

Example 5. BIOLOGICAL CHARACTERISTICS OF PEPTIDES

Cytotoxicity was tested by an indirect method based on the guidelines in ISO 10993- 5 on the hFOB human osteoblast cell line (ATCC) using the LDH assay (Roche). The results presented were averaged from two technical repeats and presented as % relative to control cells treated with 1% Triton X- 100 solution. proliferation was tested by an indirect method based on the guidelines in ISO 10993- 5 on the hFOB human osteoblast cell line (ATCC) using the WST-1 assay (Abcam). The results shown were averaged from two technical repeats and presented as % in relation to control cells cultured without added peptides.

None of the concentrations of the tested peptides induced cytotoxicity exceeding 30% (Fig. 5a, Fig. 6a).

For most concentrations of ug1/ug4 peptide, proliferation reached values above 100% of proliferation of control cells (Fig. 5b). None of the ug1/ug4 peptide concentrations resulted in proliferation lower than 70% of proliferation of control cells.

Only for peptide ug46 at a concentration of 100 $\mu$g/ml proliferation value dropped below 70%. Proliferation values exceeding 100% were observed for the other ug46 peptide titrations (Fig. 6b).

Statistical analysis, summary

Proliferation and cytotoxicity data were analysed and visualised using GraphPad Prism 8 software (GraphPad Software, USA). Statistical analysis was performed using the Kruskal- Wallis test ($p = 0.05$). In the next step, the Benjamini, Krieger and Yekutielli multiple comparisons test was performed to control the false discovery rate ($p = 0.05$). Comparisons were made between results obtained for individual peptide concentrations.

For ug1/ug4 peptides - the presence of different concentrations of ug1/ug4 peptides did not induce statistically significantly different cytotoxicity. Statistically significant differences occurred between proliferation of cells growing in the presence of 100 $\mu$g/ml and 50 $\mu$g/ml ug1/ug4 peptide ($p = 0.0331$).

For peptide ug46 - statistically significant differences occurred between the cytotoxicity of cells growing in the presence of 100 $\mu$g/ml and 25 $\mu$g/ml of peptide ug46 ($p = 0.0255$). Statistically significant differences also occurred for the comparison of the proliferation observed for peptide ug46 at 100 $\mu$g/ml with the other tested concentrations of this peptide ($p < 0 ,0001$). The tested peptides are not cytotoxic and, at appropriate concentrations, do not have a detrimental effect on cell proliferation. Peptide ug46 for most concentrations tested stimulates cell proliferation.

Example 6. ANTIMICROBIAL PROPERTIES OF ug46 PEPTIDE

The antimicrobial properties of the peptide were tested based on Clinical and Laboratory Standards Institute (CLSI) guidelines according to CLSI M07-A9 2012 using the microdilution method. Two bacterial pathogens were used for the study: Gram-positive Staphylococcus aureus PCM 2602 and Gram-negative Pseudomonas aeruginosa PCM 2563.
The bacterial inoculum for the experiment was prepared by direct inoculation of Brain-Heart Infusion medium (BHI) with bacteria from a 24-hour culture on solid medium. The density of the suspension was 0.5 on the McFarland scale. The peptide concentration analysed : 3.125 - 250 μg/ml . Serial dilutions were performed in a 96-well, flat-bottomed, hydrophobic cell culture plate using BHI liquid medium. Then the previously prepared bacterial suspension was added. The final number of pathogens in each well was approximately $5 \times 10^5$ CFU/ml (Colony Forming Units per millilitre). Microplates were incubated for 24 hours at 37 °C under aerobic conditions. The minimum inhibitory concentration (MIC) for a peptide was defined as the lowest concentration of peptide in a well where no bacterial growth was observed. All experiments were performed in triplicate.

Table 3. Minimum microbial growth inhibitory concentration values for peptide ug46 against the bacteria S. aureus PCM 2602 and P. aeruginosa PCM 2563.

| Peptide | Minimum inhibitory concentration of microorganisms (MIC) | |
| --- | --- | --- |
| | S. aureus PCM 2602 | P. aeruginosa PCM 2563 |
| ug46 | 100 μg/ml | >250 μg/ml (outside the analysed concentration range) |

Example 7. ANTI-INFLAMMATORY PROPERTIES OF ug46 PEPTIDE

The anti-inflammatory properties of peptide ug46 were tested on the human fibroblast cell line WI-38 (ATCC). Inflammation was stimulated using lipopolysaccharide (LPS) with a final concentration in the culture medium of 750 ng/ml. The level of interleukin 8 (IL-8) secreted by WI-38 cells, which is one of the markers of inflammation, was determined using the IL-8 Human Uncoated ELISA Kit (Invitrogen). Immediately before the experiment, dilutions of ug46 peptide were prepared in culture medium at concentrations of 100, 90, 80, 70, 60, 50, 40, 30, 20 and 10 μg/ml. Dilutions were made in medium with 750 ng/ml LPS and in medium without LPS. The results obtained indicate for a slight stimulation of inflammation by peptide ug46 alone at peptide concentrations above 70 μg/ml. In contrast, at peptide ug46 concentrations in the range 60-30 μg/ml, an approximately 30-50% decrease in the amount of LPS-mediated interleukin 8 was observed, indicating an anti-inflammatory effect of the peptide. In addition, at these concentrations, no deviation was observed for cells incubated without the addition of LPS (Fig. 6c).

Example 8. SYNTHESIS OF A BIOGLASS WITH SYMBOL P5_II - a bioglass containing in its composition: $SiO_2$, CaO and $P_2O_5$

One at a time, 28 ml of anhydrous ethanol was measured into a PTFE beaker and 31.2 ml of $C_8H_{20}O_4Si$ was slowly added using intense stirring with a magnetic stirrer. Then 1.44 ml of $C_8H_{20}O_5P_2$ was added catalysing the system with 2 M $HNO_3$ . After homogenisation, 28.64 g of a $Ca(NO_3)_2 \cdot 4H_2O$ 44.1 wt.% solution was added. The whole mixture was homogenised using a magnetic stirrer. The beaker with the reaction mixture was then placed in a drying oven and held at 40 °C for 7 days. After each additional 7 days, the drying temperature was increased to 60 °C, 80 °C, 120 °C and 180 °C. After holding for 7 days at 180 °C, the dried gel was transferred to a ceramic crucible and placed in an electric oven. Heating was carried out at 600° C for 10h and then at 650 °C for 15h. After removal from the furnace, the bioglass was crushed in a mechanical mortar to a grain size where 90% of the grains were below 453.260 μm. The crushed glass was then placed in a rotary-vibrating mill with 7 mm diameter alundum grinders and ground to a grain size where 90% of the grains were below 77.356 μm.
The characterisation of the P5_II bioglass was carried out according to the methodology described below. This methodology was applied to the characterisation of all bioglass (Examples 8-10).
The grain size analysis of the glass obtained was carried out with a Malvern Instruments Mastersizer 2000 laser analyser, using the low-angle laser light scattering (LALLS) method. The instrument used allows the grain size to be examined over a wide range from 0.1 μm to 2000 μm with an error of 0.5%. The following characteristic values were determined: Dv (0.1) - value of the particle size below which the is 10% of the test sample population by volume, Dv (0.5) - the particle size value below which 50% of the test sample population by volume occurs, Dv (0.9) - the particle size value below which 90% of the test sample population by volume occurs. FTIR analysis of the glass produced was performed using a Bruker TENSOR27 FTIR spectrophotometer. To prepare a suitable sample for the test (KBr tablet), a weight of approximately 200 mg of dried KBr was mixed with approximately 0.44-0.9 mg of glass sample. Measurements were performed in ATR mode. The following abbreviations were used in the description of the FTIR spectra: ν - tensile vibrations, $\nu_{as}$ - asymmetric tensile vibrations, $\nu_s$ - symmetric tensile vibrations, δ - bending vibrations. The effect of bioglass on human cells was tested using the LDH assay (Roche) cytotoxicity assay and the WST-1 assay (Abcam) cell proliferation. A human hFOB osteoblast cell line (ATCC) was exposed to 100 mg of bioglass placed on cell culture inserts (Sarstedt) (PET, translucent, pore size: 0.4 μm). Measurements were taken after 48 h of incubation of the cells with the test material. Cytotoxicity results were averaged from two technical runs and presented as % for control cells exposed to 1% Triton X-100 solution. Proliferation results were averaged from two technical runs and presented as % for control cells cultured with the insert without bioglass.

In order to test the antimicrobial effect of the bioglass, 1 ml of a bacterial suspension with an optical density of 0.5 on the McFarland scale (~$1.5 \times 10^8$ CFU/ml) was added to the 100 mg weights (in sterile 1.5 ml eppendorf tubes). The control sample was silicon (IV) oxide and the antibiotic gentamicin at a final concentration of 100 μg/ml. Each sample was agitated for 1 min using a vortex mixer. The samples were then incubated at 37 °C under aerobic conditions for 1h. After incubation, serial dilutions of each sample were made and then a volume of 100 μl of the suspension was seeded onto agar solidified LB (lysogeny broth) plates. Plates were incubated at 37 °C for 24 h. After incubation, bacterial colonies were counted and the percentage reduction in the number of microorganisms was determined according to the formula:

$$\text{Reduction of the number of micro} - \text{organisms} \ [\%] = \frac{(N_0 - N_1)}{N_0} \times 100$$

Where:

$N_0$ - number of micro-organisms for the sample without treatment with antimicrobial agent
$N_1$ - number of micro-organisms for the test with antimicrobial agent

Analytical results for bioglass P5_II

LALLS analysis results: dv(0,1) 4.214 μm; dv(0,5) 26.827 μm; dv(0,9) 77.356 μm.
FTIR analysis result: 3100-3700 cm$^{-1}$ - ν O-H (SiO-H)(HO-H), 1636 cm$^{-1}$ - δ O-H (HO-H), 1220 cm$^{-1}$ and 1070 cm$^{-1}$ - $\nu_{as}$ Si-O-Si, 798 cm$^{-1}$ - $\nu_s$ Si-O-Si, 565 and 582 cm$^{-1}$ - δ P-O in PO$_4^{3-}$, 460 cm$^{-1}$ - δ Si-O-Si.
The labelled cytotoxic effect of the bioglass is below the designated threshold of 10% (Fig. 7A). The labelled proliferation of the bioglass exceeds the designated threshold of 90% (Fig. 7B).
The determined bactericidal activity of the bioglass in contact with the micro-organisms Staphylococcus aureus PCM 2602 and Pseudomonas aeruginosa PCM 2563, in combination with gentamicin and silicon oxide, is shown in the graph (Fig. 7C).

Example 9. SYNTHESIS OF A BIOGLASS WITH SYMBOL P5Sr2_II - a bioglass containing in its composition: SiO$_2$, CaO and P$_2$O$_5$ and additionally subsidised with SrO

One at a time, 28 ml of anhydrous ethanol was measured into a PTFE beaker and 31.2 ml of C$_8$H$_{20}$O$_4$Si was slowly added using intense stirring with a magnetic stirrer. Then 1.44 ml of C$_8$H$_{20}$O$_5$P$_2$ was added catalysing the system with 2 M HNO3 . After homogenisation, 25.64 g of a Ca(NO$_3$)$_2$·4H$_2$O and 2.49 g of a Sr(NO$_3$)$_2$ solution of 19.68% w/w was added. The synthesis was then carried out as described in Example 8, except that the heating of the dried gel was carried out in an electric oven at 650 °C for 15h. After removal from the oven, the bioglass was crushed in a mechanical mortar to a grain size where 90% of the grains were below 500 μm. The crushed glass was then placed in a rotary-vibrating mill with 7 mm diameter alundum grinders and ground to a grain size where 90% of the grains were below 131.108 μm.
LALLS analysis results: dv(0,1) 5.723 μm; dv(0,5) 45.025 μm; dv(0,9) 131.108 μm.
FTIR analysis result: 3100-3700 cm$^{-1}$ - ν O-H (SiO-H)(HO-H), 1636 cm$^{-1}$ - δ O-H (HO-H), 1220 cm$^{-1}$ and 1070 cm$^{-1}$ - $\nu_{as}$ Si-O-Si, 798 cm$^{-1}$ - $\nu_s$ Si-O-Si, 565 and 582 cm$^{-1}$ - δ P-O in PO$_4^{3-}$, 460 cm$^{-1}$ - δ Si-O-Si.
The cytotoxic effect of the vitreous is below the designated threshold of 10% (Fig. 7A). Proliferation exceeds the designated threshold of 90% (Fig. 7B).
The reduction [%] in the number of micro-organisms is shown in the graph (Fig. 7C).

Example 10. SYNTHESISOF BIOGLASS WITH SYMBOL P5Zn2_II - a bioglass containing in its composition: SiO$_2$, CaO i P$_2$O$_5$ and additionally subsidised with ZnO

One at a time, 28 ml of anhydrous ethanol was measured into a PTFE beaker and 31.2 ml of C$_8$H$_{20}$O$_4$Si was slowly added using intense stirring with a magnetic stirrer. Then 1.44 ml of C$_8$H$_{20}$O$_5$P$_2$ was added catalysing the system with 2 M HNO3 . After homogenisation, 25.64 g of a Ca(NO$_3$)$_2$·4H$_2$O and 2,82 g of a Zn(NO$_3$)$_2$·6H$_2$O 29.2% w/w solution was added. The synthesis was then carried out as described in Example 8, except that the heating of the dried gel was carried out in an electric oven at 650 °C for 15 h. After removal from the oven, the bioglass was crushed in a mechanical mortar to a grain size where 90% of the grains were below 500 $\mu$m. The crushed glass was then placed in a rotary-vibrating mill with 7 mm diameter alundum grinders and ground to a grain size where 90% of the grains were below 85.620 $\mu$m.

LALLS analysis results: dv(0,1) 3.713 $\mu$m; dv(0,5) 29.058 $\mu$m; dv(0,9) 85.620 $\mu$m.

FTIR analysis result: 3100-3700 cm$^{-1}$ - v O-H (SiO-H)(HO-H), 1636 cm$^{-1}$ - $\delta$ O-H (HO-H), 1220 cm$^{-1}$ and 1070 cm$^{-1}$ - $v_{as}$ Si-O-Si, 798 cm$^{-1}$ - $v_s$ Si-O-Si, 565 and 582 cm$^{-1}$ - $\delta$ P-O in PO$_4{}^{3-}$, 460 cm$^{-1}$ - $\delta$ Si-O-Si.

The cytotoxic effect of the glasses is below the designated threshold of 10% (Fig. 7A). Proliferation exceeds the designated threshold of 90% (Fig. 7B). The reduction [%] in the number of micro-organisms is shown in the graph (Fig. 7C).

Example 11. SYNTHESIS AND CHARACTERISTICS OF CHITOSAN COMPOSITE WITH P5Zn2ll BIOGLASS AND ug4 PEPTIDE CONNECTED COVALENTIALLY TO CHITOSAN CHAIN IN A 0.8% SIDE (CH_CH_ug4_0.8_P5Zn2ll).

To 0.4 g of chitosan, 0.0354 g of chitosan modified with ug4 peptide and 16.9806 g of 1% (v/v) acetic acid were added, and the contents of the vessel were stirred until the chitosans were completely dissolved. At the same time, 0.4354 g of P5Zn2_II bioglass and 4.354 g of 1% (v/v) acetic acid were placed in a separate vessel and stirred until a homogeneous paste of bioglass particles was formed. The resulting paste was then introduced into a vessel with the chitosan solution and stirred until a stable dispersion of the bioglass particles in the acidic chitosan solution was obtained. The suspension produced was transferred to a mould of the desired shape and size and subjected to the freeze-drying process. After removal from the freeze-dryer chamber, the dried materials were protected against moisture. The resulting composite porous scaffolds were stabilised by immersion in 96% ethanol for 6 hours. After this time, the stabilised scaffolds were washed with 0.1 M NaOH for 1 hour and then with deionised water for 24 hours, changing the water several times until the pH was in the range of 6.5-7.5. The washed scaffolds were subjected to freeze-drying again. The final product had a chitosan/bioglass content of 1/1, with 8.13 wt.% chitosan as modified chitosan containing 55.57 wt.% of ug4 peptide.

The characterisation of the CH_CH_ug4_0.8_P5Zn2II composite was carried out according to the methodology described below. This methodology was also applied to the characterisation of the porous composites in the following examples 12 - 20.

The porous microstructure of the composite and its pore size range were confirmed by scanning electron microscopy SEM. The specific surface area was determined using the BET method. The product structure was confirmed by Fourier transform infrared spectroscopy. The bioactivity of the composite, defined as the growth of an apatite layer on the composite surface, was confirmed by SEM observations and EDS analysis of composites incubated in simulated body fluid (SBF) at 37 °C for 4 weeks. Cytotoxicity was tested by an indirect method based on guidelines from ISO 10993-5 on a human hFOB osteoblast cell line (ATCC) using an LDH assay (Roche). Results shown were averaged from two technical runs and presented as % relative to control cells treated with 1% Triton X-100 solution. Proliferation was tested by an indirect method based on guidelines from ISO 10993-5 on a human osteoblast cell line hFOB (ATCC) using the WST-1 assay (Abcam). The results shown were averaged from two technical runs and presented as % relative to control cells not in contact with the composite extract. Peptide release from the composite was determined by immersing the composite in water with MMP-7 enzyme and analysing the compounds released into solution over time, qualitative and quantitative analysis was performed by UPLC and mass spectrometry. Release experiments were performed in a 24-well plate at 37 °C with continuous shaking. The discs were placed in the well of the plate and 1 ml of deionised water and 1 $\mu$l of MMP-7 enzyme were added. At each time point, 65 $\mu$l of the sample solution was taken and the volume taken was replenished with 65 $\mu$l of deionised water. Performed over a time interval of 0 - 48h, 2 discs were used for the experiment. High-performance reversed-phase liquid chromatography (Nexera X2, Shimadzu) was used to quantify the peptide content of the samples using an analytical Kromasil column (4.6 × 250 mm; C-8; 5 $\mu$m), the volumetric flow rate was 0.5 ml/min, eluents: A - 0.1% TFA in H$_2$O (v/v), B- 80% CH$_3$ CN in 0.1% TFA in H$_2$O (v/v). Qualitative analysis was performed using a Bruker Briflex III MALDI-TOF spectrometer from Bruker Daltonics.

SEM, SEM/EDS: The resulting composite has a porous structure with a pore size in the range 43.36-121.97 $\mu$m (Fig. 8a).

BET: The specific surface area of the composite was 44.1454 $\pm$ 0.0698 [m$^2$/g].

FTIR-ATR: 3364-3186 cm$^{-1}$ and 3453 cm$^{-1}$ - v OH, v NH, 2910 cm$^{-1}$, 2864 cm$^{-1}$ - v CH, 1647 cm$^{-1}$ - $v$ C=O, 1582 cm$^{-1}$ - $\delta$ N-H, 1463 cm$^{-1}$-1261 cm$^{-1}$- $\delta$ - OH, $\delta$ C-H and v C-O w (-CH$_2$-OH) and v C=N, 1151 cm$^{-1}$ - $v_{as}$ C-O-C, 1069 cm$^{-1}$ and 1032 cm$^{-1}$ - $v_{as}$ Si-O-Si, 895 cm$^{-1}$ - v C-O-C, 717 cm$^{-1}$ - $\delta$ O=CN (in ug4) and 664 cm$^{-1}$ and 605 cm$^{-1}$ - $\delta$ P-O.

Bioactivity: EDS analysis of the surface of the composite incubated in SBF for 4 weeks showed the presence of intense Ca and P signals, indicating that an additional layer of calcium phosphate is formed on the surface of the incubated composite and that the composite therefore exhibits bioactivity. The SEM image of the incubated composite shows characteristic hydroxyapatite islands (Fig. 9a).

Cytotoxicity: the mean cytotoxicity of the composite was -5.623% ($\pm$ 3.157%) relative to control cells. The composite showed no cytotoxic properties (Fig. 10A).

Proliferation: the average proliferation of the composite was 120% ($\pm$ 6.075%) relative to control cells. The composite showed pro-proliferative properties (Fig. 10B).

Peptide release: the amount of peptide released was below the limit of quantification.

EXAMPLE 12. SYNTHESIS AND CHARACTERISTICS OF CHITOSAN COMPOSITE WITH P5Zn2ll BIOGLASS AND ug4 PEPTIDE CONNECTED COVALENTIALLY TO CHITOSAN CHAIN IN A 1.6% SIDE (CH_CH_ug4_1.6_P5Zn2ll).

To 0.4 g of chitosan, 0.078 g of chitosan modified with ug4 peptide and 18.642 g of 1% (v/v) acetic acid were added, and the contents of the vessel were stirred until the chitosans were completely dissolved. At the same time, 0.478 g of P5Zn2_II bioglass and 4.78 g of 1% (v/v) acetic acid were placed in a separate vessel and stirred until a homogeneous paste was formed. The process of obtaining the composite was then carried out identically to Example 11. In the final product, the content of chitosan relative to the bioglass was 1/1, with 16.32 wt.% chitosan being a modified chitosan containing 55.57 wt.% of ug4 peptide.

SEM, SEM/EDS: The resulting composite has a porous structure with a pore size in the range 70.9-130.12 $\mu$m (Fig. 8b).

BET: The specific surface area of the composite was 62.5828 $\pm$ 0.1343 [m$^2$/g].

FTIR-ATR: 3363-3181 cm$^{-1}$ and 3453 cm$^{-1}$ - v OH, v NH, 2911 cm$^{-1}$, 2874 cm$^{-1}$ - v CH, 1642 cm$^{-1}$ - $\nu$ C=O, 1581 cm$^{-1}$ - $\delta$ N-H, 1461 cm$^{-1}$-1251 cm$^{-1}$ - $\delta$ - OH, $\delta$ C-H and v C-O in (-CH$_2$-OH) and v C=N, 1154 cm$^{-1}$ - $\nu_{as}$ C-O-C, 1069 cm$^{-1}$ and 1032 cm$^{-1}$ - $\nu_{as}$ Si-O-Si, 895 cm$^{-1}$ - v C-O-C, 720 cm$^{-1}$ - $\delta$ O=CN (in ug4) and 661 cm$^{-1}$ and 605 cm$^{-1}$ - $\delta$ P-O.

Bioactivity: The composite shows bioactivity after 2 weeks of incubation in SBF (Fig. 9b). Cytotoxicity: The average cytotoxicity of the composite was -3.602% ($\pm$ 3.169%) relative to control cells. The composite showed no cytotoxic properties (Fig. 10A).

Proliferation: the average proliferation of the composite was 116.8% ($\pm$ 4.606%) relative to control cells. The composite showed pro-proliferative properties (Fig. 10B).

Peptide release: the amount of peptide released was below the limit of quantification.

EXAMPLE 13. SYNTHESIS AND CHARACTERISTICS OF CHITOSAN COMPOSITE WITH P5Zn2ll BIOGLASS AND ug4 PEPTIDE CONNECTED COVALENTIALLY TO CHITOSAN CHAIN IN A 3.4% SIDE (CH_CH_ug4_3,4_P5Zn2ll).

To 0.4 g of chitosan, 0.15 g of chitosan modified with ug4 peptide and 21.45 g of 1% (v/v) acetic acid were added, and the contents of the vessel were stirred until the chitosans were completely dissolved. At the same time, 0.55 g of P5Zn2_II bioglass and 5.5 g of 1% (v/v) acetic acid were placed in a separate vessel and stirred until a homogeneous paste was formed. The process of obtaining the composite was then carried out identically to Example 11. In the final product, the content of chitosan relative to the bioglass was 1/1, with 27.27 wt.% chitosan being a modified chitosan containing 55.57 wt.% of ug4 peptide.

SEM, SEM/EDS: The resulting composite has a porous structure with a pore size in the range 73.4-161.29 $\mu$m (Fig. 8c).

BET: The specific surface area of the composite was 34.8540 $\pm$ 0.1727 [m$^2$/g].

FTIR-ATR: 3353-3181 cm$^{-1}$ and 3453 cm$^{-1}$ - v OH, v NH, 2909 cm$^{-1}$, 2872 cm$^{-1}$ - v CH, 1647 cm$^{-1}$ - $\nu$ C=O, 1582 cm$^{-1}$ - $\delta$ N-H, 1496 cm$^{-1}$-1253 cm$^{-1}$ - $\delta$ - OH, $\delta$ C-H and v C-O w (-CH$_2$-OH) and v C=N, 1151 cm$^{-1}$ - $\nu_{as}$ C-O-C, 1069 cm$^{-1}$ and 1032 cm$^{-1}$ - $\nu_{as}$ Si-O-Si, 895 cm$^{-1}$ - v C-O-C, 712 cm$^{-1}$ - $\delta$ O=CN (in ug4) and 654 cm$^{-1}$ and 605 cm$^{-1}$ - $\delta$ P-O.

Bioactivity: The composite shows bioactivity after 3 weeks of incubation in SBF (Fig. 9c). Cytotoxicity: The average cytotoxicity of the composite was -2.446% ($\pm$ 3.138%) relative to control cells. The composite showed no cytotoxic properties (Fig. 10A).

Proliferation: the average proliferation of the composite was 187.1% ($\pm$ 9.894%) relative to control cells. The composite showed pro-proliferative properties (Fig. 10B).

Peptide release: the amount of peptide released was below the limit of quantification.

EXAMPLE 14. SYNTHESIS AND CHARACTERISTICS OF CHITOSAN COMPOSITE WITH P5Sr2ll BIOGLASS AND ug4 PEPTYD CONNECTED COVALENTIALLY TO CHITOSAN CHAIN IN A 3.4% BACKGROUND (CH_CH_ug4_3,4_P5Sr2ll).

To 0.4 g of chitosan, 0.15 g of chitosan modified with ug4 peptide and 21.45 g of 1% (v/v) acetic acid were added, and the contents of the vessel were stirred until the chitosans were completely dissolved. At the same time, 0.55 g of P5Sr2_II bioglass and 5.5 g of 1% (v/v) acetic acid were placed in a separate vessel and stirred until a homogeneous paste was formed. The process of obtaining the composite was then carried out identically to Example 11. In the final product, the content of chitosan relative to the bioglass was 1/1, with 27.27 wt.% chitosan being a modified chitosan containing 55.57 wt.% of ug4 peptide.

SEM, SEM/EDS: The resulting composite has a porous structure with a pore size in the range 38.96-107.24 $\mu$m (Fig. 8d).

BET: The specific surface area of the composite was 31.6170 $\pm$ 0.3757 [m$^2$/g].

FTIR-ATR: 3363-3181 cm$^{-1}$ and 3454 cm$^{-1}$ - v OH, v NH, 2909 cm$^{-1}$, 2864 cm$^{-1}$ - v CH, 1649 cm$^{-1}$ - v C=O, 1582 cm$^{-1}$ - $\delta$ N-H, 1460 cm$^{-1}$-1262 cm$^{-1}$ - $\delta$ - OH, $\delta$ C-H and v C-O in (-CH$_2$-OH) and v C=N, 1151 cm$^{-1}$ - $\nu_{as}$ C-O-C, 1070 cm$^{-1}$ and 1032 cm$^{-1}$ - $\nu_{as}$ Si-O-Si, 895 cm$^{-1}$ - v C-O-C, 710 cm$^{-1}$ - $\delta$ O=CN (in ug4) and 664 cm$^{-1}$ and 606 cm$^{-1}$ - $\delta$ P-O.

Bioactivity: The composite shows bioactivity after 1 week of incubation in SBF (Fig. 9d). Cytotoxicity: The average cytotoxicity of the composite was -6.160% ($\pm$ 1.026%) relative to control cells. The composite showed no cytotoxic properties (Fig. 10A).

Proliferation: the average proliferation of the composite was 96.07% ($\pm$ 4.563%) relative to control cells. The composite showed pro-proliferative properties (Fig. 10B).

Release of peptide: Released amount of peptide was found located below the limit of quantification.

EXAMPLE 15. SYNTHESIS AND CHARACTERISTICS OF CHITOSAN COMPOSITE WITH P5Zn2II BIOGLASS AND ug4 PEPTIDE CONNECTED COVALENTIALLY TO CHITOSAN CHAIN AT 3.4% SURFACE AND ug46 PEPTIDE ADSORBATED ON THE COMPOSITE SURFACE (CH_CH_ug4_3,4_P5Zn2II_ug46).

To 0.4 g of chitosan, 0.15 g of chitosan modified with ug4 peptide and 21.45 g of 1% (v/v) acetic acid were added, and the contents of the vessel were stirred until the chitosans were completely dissolved. At the same time, 0.55 g of P5Zn2_II bioglass and 5.5 g of 1% (v/v) acetic acid were placed in a separate vessel and stirred until a homogeneous paste was formed and the process of obtaining the composite was continued according to the method described in Example 11. The porous composite produced was then immersed in an aqueous solution of ug46 peptide at a concentration of 0.29 mg/ml for 24 h, after which it was freeze-dried again. The final product had a chitosan/bioglass content of 1/1, with 27.27 wt.% chitosan as modified chitosan containing 55.57 wt.% of ug4 peptide.

SEM, SEM/EDS: The resulting composite has a porous structure with a pore size in the range 53.63-131.98 $\mu$m (Fig. 8e).

BET: The specific surface area of the composite was 62.6129 $\pm$ 0.1286 [m$^2$/g].

FTIR-ATR: 3363-3191 cm$^{-1}$ and 3453 cm$^{-1}$ - v OH, v NH, 2911 cm$^{-1}$, 2864 cm$^{-1}$ - v CH, 1647 cm$^{-1}$ - v C=O, 1582 cm$^{-1}$ - $\delta$ N-H, 1466 cm$^{-1}$-1259 cm$^{-1}$ - $\delta$ - OH, $\delta$ C-H and v C-O in (-CH$_2$-OH) and v C=N, 1151 cm$^{-1}$ - $\nu_{as}$ C-O-C, 1069 cm$^{-1}$ and 1032 cm$^{-1}$ - $\nu_{as}$ Si-O-Si, 895 cm$^{-1}$ - v C-O-C, 710 cm$^{-1}$ - $\delta$ O=CN (peptides) and 665 cm$^{-1}$ and 605 cm$^{-1}$ - $\delta$ P-O.

Bioactivity: The composite shows bioactivity after 2 weeks of incubation in SBF (Fig. 9e). Cytotoxicity: The average cytotoxicity of the composite was 1.202% ($\pm$ 2.527%) against control cells. The composite showed no cytotoxic properties (Fig. 10A).

Proliferation: the average proliferation of the composite was 178.1% ($\pm$ 11.58%) relative to control cells. The composite showed pro-proliferative properties (Fig. 10B).

Release of peptide: Released amount of peptide was found located below the limit of quantification.

For the composite CH_CH_ug4_3.4_P5Zn2II_ug46, and other composites enriched with the ug46 peptide (Examples 16-20), the antimicrobial surface activity was determined according to the adapted ASTM E2180-07 2012 standard using the Gram-positive bacterium Staphylococcus aureus PCM 2602 and the Gram-negative bacterium Pseudomonas aeruginosa PCM 2563 against a control sample - a composite without the addition of the bioglass/peptide (CH).

Antimicrobial activity: The average percentage reduction in the number of bacteria relative to the control sample (CH) was 95.00% against S. aureus bacteria and 86.41% against P. aeruginonosa bacteria (Fig. 11).

For the CH_CH_ug4_3.4_P5Zn2II_ug46 composite, in vivo biocompatibility was tested. The tests were performed in accordance with EN ISO 10993-6: "Biological evaluation of medical devices - Part 6: Tests for local effects after implantation" and EN ISO 10993-11: "Biological evaluation of medical devices - Part 11: Tests for systemic toxicity".

Tests for systemic toxicity in vivo.

The study was performed according to the recommendations of EN ISO 10993-11: "Biological evaluation of medical devices - Part 11: Tests for systemic toxicity". Samples of the CH_CH_ug4_3.4_P5Zn2II_ug46 composite were formed into 10 mm $\times$ 0.5 mm discs and subjected to radiation sterilisation. A systemic acute toxicity study was carried out on 10 albino Swiss strain, male mice, allocating 5 animals to the biocomposite study and 5 animals to the control group. The initial mean body weight of the animals was 35.68 ($\pm$1.80) g in the control group and 37.3($\pm$3.11) g in the group allocated to the composite study. The animals, housed in groups, were subjected to handling training during the acclimatisation period in order to become accustomed to the touch of the investigator. Tests were carried out after intraperitoneal injection of aqueous extracts from the test biocomposite at a rate of 50 ml/kg of mouse body weight (i.e. from 1.62 ml to 2.06 ml, averaging 1.75 ml for individual mice) in the test group and similarly-injection from saline for injection in the control group. The extract of the tested biocomposite was prepared after flooding the samples - with saline solution for injection at a ratio of the bilateral surface area of the samples to the volume of the extraction mixture - 3 $cm^2$/ml. The extracts were then incubated with a parallel control, which was saline - solution for injection at 37 °C for 72 h. Observations of the injected animals were carried out for 7 days and included changes in appearance, behavioural abnormalities, stunted weight gain and mortality. Mice were weighed daily, normal function was observed, feed and water consumption was measured, and differences in weight gain between animals of the test and control groups were assessed.

No physical or behavioural changes indicative of disease were found in either the test group or the control group during the health observations carried out on the animals. Each physical examination considered the test animal to be clinically healthy. During the 7-day experimental (post-inoculation) period, there were no behavioural deviations or any signs of disease in the animals, either in the control group or the test group. All mice were considered clinically healthy. Feed and water consumption and body weight gain in the test groups were comparable to the data obtained in the control group, and the differences were not statistically significant. All mice survived until the scheduled postmortem date. There were no abnormalities in behaviour, appearance or differences in weight gain between the test groups and the control group. After 7 days, euthanasia (with pentobarbital) and postmortem examinations were performed. Postmortem examinations of the animals, both from the control group and the test group, revealed no skin lesions, skeletal or muscular changes. The natural body orifices (external ear, eyes, nostrils, mouth, anus) and external genitalia showed no lesions. In the peritoneal cavity and thorax, all organs were positioned correctly, with preserved anatomical shape, colour and size. None of the visually inspected organs showed macroscopic pathological changes. No pathological changes were found at autopsy. On the basis of tests carried out on the basis of EN ISO 10993-11: "Biological evaluation of medical devices - Part 11: Tests for systemic toxicity", it was concluded that the samples of the tested biocomposite do not show systemic acute toxicity.

Post-implantation local response studies.

For the CH_CH_ug4_3.4_P5Zn2ll_ug46 composite, in vivo biocompatibility was tested using a rabbit model (New Zealand rabbits, both sexes). The tests were performed in accordance with EN ISO 10993-6: "Biological evaluation of medical devices - Part 6: Tests for local effects after implantation" and EN ISO 10993-11: "Biological evaluation of medical devices - Part 11: Tests for systemic toxicity" (bioethics committee approval for experiments number 83/2019/P1). The study plan included: implantation into the femur of rabbits (2 implants were placed in each trochanter), macroscopic (postmortem) and histological examinations at 1, 2 and 3 months after surgery, and radiological examinations. After implantation of the tested composite, observation of the rabbits' general health was carried out, with particular consideration on postoperative wound healing, active and passive mobility of the hip joint and feed intake. During the dissections performed, the post- operative wound and the appearance of the tissues at the site of implantation of the specimens were evaluated macroscopically first, followed by an assessment of the appearance of selected internal organs. Subsequently, femurs with implants were collected for further histological and radiological examination.

After implantation of the CH_CH_ug4_3,4_P5Zn2II_ug46 composite at 1-month to 3-month follow-up, the condition of the animals did not deviate from normal. The animals retained active and passive mobility in the hip joints. Postoperative wounds healed by rapid growth. Early in the post-operative examination, a slight protrusion containing small amounts of serous fluid was found in the wound area, which disappeared after 3-4 days and the wounds healed normally. On microscopic examination, the composite was visible up to day 90 after implantation. Histological examination at 1 month after implantation revealed a band of loose connective tissue that separated the implant from the surrounding cancellous bone tissue. In the immediate vicinity in the bone, high osteoblast activity was visible, and in the connective tissue band inflammatory granulation tissue associated with regenerative processes was present (Fig. 12a) . At 60 and 90 day after implantation, the indentation process was similar to the previous study period. The implants remained visible undergoing gradual reduction and deformation. In the centre of the implants, foci of mineralisation were more numerous than in the previous study period. On radiographic imaging, the surgically performed cavities remained filled with ossein, the biocomposite remained invisible. No osteosclerotic reactions were found at the implant site, and there were no abnormal changes in the bone structure (Fig. 13a).

Example 16. SYNTHESIS AND CHARACTERISTICS OF CHITOSAN COMPOSITE WITH P5Zn2ll BIOGLASS ADDED AND PEPTIDE INTRODUCED IN THE FORM OF UG46 FIBRILS IN AN AMOUNT OF 0.17 WT.%. OF THE COMPOSITE (CH_P5Zn2II_UG-46 (1))

To 0.75 g of chitosan, 29.25 g of 1% (v/v) acetic acid was added and the contents of the vessel were stirred until the chitosan was completely dissolved. At the same time, 0.75 g of P5Zn2_II bioglass and 7.5 g of 1% (v/v) acetic acid were placed in a separate vessel and stirred until a homogeneous paste of bioglass particles was formed. The resulting paste was then introduced into a vessel with chitosan solution and stirred until a stable dispersion of the bioglass particles in the acidic chitosan solution was obtained. A 3 mL aqueous solution of UG46 peptide fibrils (including 2.5 mg UG46) was then added and the whole mass was stirred for 10 min. The suspension produced was transferred into a mould of the desired shape and size and lyophilised. The composite porous scaffolds obtained by freeze-drying were stabilised by immersion in 96% ethanol for 6 hours. After this time, the stabilised scaffolds were washed with 0.1 M NaOH for 1 hour and then with deionised water for 24 hours, changing the water several times until the pH was in the range of 6.5-7.5. The washed scaffolds were subjected to freeze-drying again. SEM, SEM/EDS: The resulting composite has a porous structure with a pore size in the range of 41.06-91.6 $\mu$m (Fig. 14a).

BET: The specific surface area of the composite is 59.9800 $\pm$ 0.1251 [m$^2$/g].

FTIR-ATR: 3453-3188 cm$^{-1}$ - v OH, v NH, 2909 cm$^{-1}$, 2865 cm$^{-1}$ - v CH, 1647 cm$^{-1}$ - v C=O, 1581 cm$^{-1}$ - $\delta$ N-H, 1461 cm$^{-1}$-1260 cm$^{-1}$ - $\delta$ - OH, $\delta$ C-H and v C-O w (-CH$_2$-OH) and v C=N, 1151 cm$^{-1}$ - $v_{as}$ C-O-C, 1070 cm$^{-1}$ and 1032 cm$^{-1}$ - $v_{as}$ Si-O-Si, 895 cm$^{-1}$ - v C-O-C, 712 cm$^{-1}$ - $\delta$ O=CN (UG46) and 665 cm$^{-1}$ and 606 cm$^{-1}$ - $\delta$ P-O.

Bioactivity: the composite shows bioactivity after 2 weeks of incubation in SBF (Fig. 15a). Cytotoxicity: mean cytotoxicity was 21.77% ($\pm$ 9.833%) relative to control cells (Fig. 16a). Proliferation: mean proliferation was 164.3% ($\pm$ 44.67%) relative to control cells (Fig. 16b).

Antimicrobial activity: The average percentage reduction in the number of bacteria relative to the control sample was 86.19% against S. aureus bacteria and 82.56% against P. aerugionosa bacteria (Fig. 11).

For the CH_P5Zn2II_UG-46(1) composite, in vivo biocompatibility was also tested according to the methodology described in Example 15, except that the baseline mean body weight of mice in the acute toxicity test was 35.68 ($\pm$1.80) g in the control group and 36.32 ($\pm$5.05) g in the test group.

During the acute toxicity tests, no physical or behavioural changes indicative of disease were found in either the test group or the control group during the health observations carried out on the animals. Each physical examination considered the test animal to be clinically healthy. During the 7-day experimental (post-inoculation) period, there were no behavioural deviations or any signs of disease in the animals, either in the control group or the test group. All mice were considered clinically healthy. Feed and water consumption and body weight gain in the test groups were comparable to the data obtained in the control group, and the differences were not statistically significant. All mice survived until the scheduled postmortem date. There were no abnormalities in behaviour, appearance or differences in weight gain between the test groups and the control group. After 7 days, euthanasia (with pentobarbital) and postmortem examinations were performed. Postmortem examinations of the animals, both from the control group and the test group, revealed no skin lesions, skeletal or muscular changes. The natural body orifices (external ear, eyes, nostrils, mouth, anus) and external genitalia showed no lesions. In the peritoneal cavity and thorax, all organs were positioned correctly, with preserved anatomical shape, colour and size. None of the visually inspected organs showed macroscopic pathological changes. No pathological changes were found at autopsy. On the basis of tests carried out on the basis of EN ISO 10993-11: "Biological evaluation of medical devices - Part 11: Tests for systemic toxicity", it was concluded that the samples of the tested biocomposite do not show systemic acute toxicity.

When examining the post-implantation local response of the CH_P5Zn2II_UG-46(1) composite at 1-month to 3-month follow-up, the condition of the animals did not deviate from normal. The animals retained active and passive mobility in the hip joints. Post-operative wounds healed by rapid growth. Early in the post-operative examination, a slight protrusion containing small amounts of serous fluid was found in the wound area, which disappeared after 3-4 days and the wounds healed normally. On microscopic examination, the composite was visible up to 90 days after implantation. Histological examination at 1 month after implantation revealed a band of loose connective tissue that separated the implant from the surrounding cancellous bone tissue. In the immediate vicinity in the bone, osteoblast activity was visible slightly lower than in the composite of example 15, and in the connective tissue band inflammatory granulation associated with regenerative processes was visible (Fig. 12b). At 60 and 90 days after implantation, the indentation process was similar to the previous study period. The implants remained visible undergoing gradual reduction and deformation. In the centre of the implants, foci of mineralisation were more numerous than in the previous study period. On radiographic imaging, the surgically performed cavities remained filled with ossein, the biocomposite remained invisible. A formed ossin was found at the implant site, in one case an osteolytic reaction was visible around the CH_P5Zn2II_UG- 46 (1) composite implant after 60 days. Other than that, no osteosclerotic reactions were found, and no abnormal changes in the bone structure were observed (Fig. 13b).

Example 17. SYNTHESIS AND CHARACTERISTICS OF CHITOSAN COMPOSITE WITH P5Zn2II BIOGLASS ADDED AND PEPTIDE INTRODUCED IN THE FORM OF UG46 FIBRILS IN AN AMOUNT OF 0.33 WT.% OF THE COMPOSITE (CH_P5Zn2II_UG-46 (2))

The process of obtaining the composite was carried out as described in Example 16, except that aqueous solutions of peptide fibrils were added to the stable dispersion of the bioglass in acidic chitosan solution in an amount of 6 ml (including 5 mg of UG46) and the process was again continued as described in Example 16.

SEM, SEM/EDS: The resulting composite has a porous structure with a pore size in the range of 54.51-117.38 $\mu$m (Fig. 14b).

FTIR-ATR: 3453-3191 cm$^{-1}$ - v OH, v NH, 2912 cm$^{-1}$, 2864 cm$^{-1}$ - v CH, 1594 cm$^{-1}$ - v C=O, 1562 cm$^{-1}$ - $\delta$ N-H, 1463 cm$^{-1}$-1263 cm$^{-1}$ - $\delta$ - OH, $\delta$ C-H and v C-O w (-CH$_2$-OH) and v C=N, 1151 cm$^{-1}$ - $v_{as}$ C-O-C, 1069 cm$^{-1}$ and 1032 cm$^{-1}$ - $v_{as}$ Si-O-Si, 895 cm$^{-1}$ - v C-O-C, 712 cm$^{-1}$ - $\delta$ O=CN (UG46) and 664 cm$^{-1}$ and 605 cm$^{-1}$ - $\delta$ P-O.

Bioactivity: the composite shows bioactivity after 3 weeks of incubation in SBF (Fig. 15b).

Cytotoxicity: the mean cytotoxicity was 26.25% ($\pm$ 5.653%) against control cells (Fig. 16a).

Proliferation: mean proliferation was 136.8% ($\pm$ 53.35%) relative to control cells (Fig. 16b).

Antimicrobial activity: The average percentage reduction in the number of bacteria relative to the control sample was 94.76% against S. aureus bacteria and 83.33% against P. aerugionosa bacteria (Fig. 11).

Example 18. SYNTHESIS AND CHARACTERISTICS OF CHITOSAN COMPOSITE WITH P5Zn2ll BIOGLASS ADDED AND PEPTIDE INTRODUCED IN THE FORM OF UG46 FIBRILS IN AN AMOUNT OF 0.5 WT.% OF THE COMPOSITE (CH_P5Zn2II_UG-46 (3))

The process of obtaining the composite was carried out as described in Example 16, except that aqueous solutions of peptide fibrils were added to the stable dispersion of the bioglass in acidic chitosan solution in an amount of 9 ml (including 7.5 mg of UG46) and the process was again continued as described in Example 16.

SEM, SEM/EDS: The resulting composite has a porous structure with a pore size in the range of 54.51-117.38 $\mu$m (Fig. 14c).

BET: The specific surface area of the composite is 66.89-138.08 [m$^2$/g].

FTIR-ATR: 3453-3186 cm$^{-1}$ - v OH, v NH, 2911 cm$^{-1}$, 2864 cm$^{-1}$ - v CH, 1644 cm$^{-1}$ - v C=O, 1565 cm$^{-1}$ - $\delta$ N-H, 1461 cm$^{-1}$-1251 cm$^{-1}$ - $\delta$ - OH, $\delta$ C-H and v C-O w (-CH$_2$-OH) and v C=N, 1151 cm$^{-1}$ - $v_{as}$ C-O-C, 1069 cm$^{-1}$ and 1032 cm$^{-1}$ - $v_{as}$ Si-O-Si, 895 cm$^{-1}$ - v C-O-C, 712 cm$^{-1}$ - $\delta$ O=CN (UG46) and 665 cm$^{-1}$ and 605 cm$^{-1}$ - $\delta$ P-O.

Bioactivity: the composite shows bioactivity after 1 week of incubation in SBF (Fig. 15c).

Cytotoxicity: mean cytotoxicity was 14.13% ($\pm$ 13.05%) relative to control cells (Fig. 16a). Proliferation: mean proliferation was 107.5% ($\pm$ 37.25%) relative to control cells (Fig. 16b).

Antimicrobial activity: The average percentage reduction in the number of bacteria relative to the control sample was 94.76% against S. aureus bacteria and 83.33% against P. aerugionosa bacteria (Fig. 11).

Example 19. SYNTHESIS AND CHARACTERISTICS OF CHITOSAN COMPOSITE WITH P5Sr2II BIOGLASS ADDED AND A PEPTIDE INTRODUCED IN THE FORM OF UG46 FIBRILS IN AN AMOUNT OF 0.17 WT.% OF THE COMPOSITE (CH_P5Sr2II_UG-46 (1))

To 0.75 g of chitosan, 29.25 g of 1% (v/v) acetic acid was added and the contents of the vessel were stirred until the chitosan was completely dissolved. At the same time, 0.75 g of P5Sr2_II bioglass and 7.5 g of 1% (v/v) acetic acid were placed in a separate vessel and stirred until a homogeneous paste of bioglass particles was formed. The process to obtain the composite was then carried out identically as in Example 16 and to the stable dispersion of the bioglass in the acidic chitosan solution, aqueous solutions of peptide fibrils were added in an amount of 3 mL (including 2.5 mg of UG46) and the process was again continued as described in Example 16.

SEM, SEM/EDS: The resulting composite has a porous structure with a pore size in the range of 51.1-92.8 $\mu$m (Fig. 14d).

BET: The specific surface area of the composite is 72.7594 $\pm$ 0.1300 [m$^2$/g].

FTIR-ATR: 3453-3191 cm$^{-1}$ - v OH, v NH, 2909 cm$^{-1}$, 2864 cm$^{-1}$ - v CH, 1652 cm$^{-1}$ - v C=O, 1562 cm$^{-1}$ - $\delta$ N-H, 1450 cm$^{-1}$ -1262 cm$^{-1}$ - $\delta$ - OH, $\delta$ C-H and v C-O w (-CH$_2$-OH) and v C=N, 1151 cm$^{-1}$ - $v_{as}$ C-O-C, 1071 cm$^{-1}$ and 1032 cm$^{-1}$ - $v_{as}$ Si-O-Si, 895 cm$^{-1}$ - v C-O-C, 712 cm$^{-1}$ - $\delta$ O=CN (UG46) and 664 cm$^{-1}$ and 605 cm$^{-1}$ - $\delta$ P-O.

Bioactivity: the composite shows bioactivity after 1 week of incubation in SBF (Fig. 15d). Cytotoxicity: the mean cytotoxicity was 10.60% ($\pm$ 12.54%) against control cells (Fig. 16a).

Proliferation: mean proliferation was 138.8% ($\pm$ 32.48%) relative to control cells (Fig. 16b).

Antimicrobial activity: The average percentage reduction in the number of bacteria relative to the control sample was 64.76% against S. aureus bacteria and 65.90% against P. aerugionosa bacteria (Fig. 11).

Example 20. SYNTHESIS AND CHARACTERISTICS OF CHITOSAN COMPOSITE WITH P5Zn2ll BIOGLASS ADDED AND ug46 PEPTIDE ADDED ON THE COMPOSITE SURFACE (CH_P5Zn2II_ug46).

To 0.75 g of chitosan, 29.25 g of 1% (v/v) acetic acid was added and the contents of the vessel were stirred until the chitosan was completely dissolved. At the same time, 0.75 g of P5Zn2_II bioglass and 7.5 g of 1% (v/v) acetic acid were placed in a separate vessel and stirred until a homogeneous paste of bioglass particles was formed. The resulting paste was then introduced into a vessel with the chitosan solution and stirred until a stable dispersion of the bioglass particles in the acidic chitosan solution was obtained. The suspension produced was transferred into a mould of the desired shape and size and lyophilised. After removal from the freeze-dryer chamber, the dried materials were protected against moisture. The resulting composite porous scaffolds were stabilised by immersion in 96% ethanol for 6 hours. After this time, the stabilised scaffolds were washed with 0.1 M NaOH for 1 hour and then with deionised water for 24 hours, changing the water several times until the pH was in the range of 6.5-7.5. The washed scaffolds were subjected to freeze-drying again. The dry porous composites were then immersed in an aqueous solution of ug46 peptide at a concentration of 0.29 mg/ml for 24 h and freeze-dried again. The final product had a chitosan/bioglass content of 1/1 and the amount of ug46 peptide adsorbed on the composite was 5.34 mg peptide /1 g composite.

SEM, SEM/EDS: The resulting composite has a porous structure with a pore size in the range of 64.65-175.15 $\mu$m (Fig. 14e).

FTIR-ATR: 3453-3191 cm$^{-1}$ - v OH, v NH, 2909 cm$^{-1}$, 2864 cm$^{-1}$ - v CH, 1642 cm$^{-1}$ - v C=O, 1582 cm$^{-1}$ - $\delta$ N-H, 1459 cm$^{-1}$-1258 cm$^{-1}$ - $\delta$ - OH, $\delta$ C-H and v C-O w (-CH$_2$-OH) and v C=N, 1151 cm$^{-1}$ - $v_{as}$ C-O-C, 1069 cm$^{-1}$ and 1032 cm$^{-1}$ - $v_{as}$ Si-O-Si, 895 cm$^{-1}$ - v C-O-C, 712 cm$^{-1}$ - $\delta$ O=CN (ug46) and 664 cm$^{-1}$ and 605 cm$^{-1}$ - $\delta$ P-O.

Bioactivity: The composite shows bioactivity after 3 weeks of incubation in SBF (Fig. 15e).

Cytotoxicity: The average cytotoxicity of the composite was -9.018% ($\pm$ 5.328%) relative to control cells. The composite showed no cytotoxic properties (Fig. 16a).

Proliferation: the average proliferation of the composite was 145.2% ($\pm$ 2.628%) relative to control cells. The composite showed pro-proliferative properties (Fig. 16b).

Antimicrobial activity: the average percentage reduction in the number of bacteria relative to the control sample (CH) was 90.62% against Staphylococcus aureus and 81.28% against Pseudomonas aerugionosa (Fig. 11).

Peptide release: the amount of peptide released was below the limit of quantification.

ug4 peptide sequence - SEQ1

<110> Lukasiewicz Research Network - Institute of Ceramics and Building Materials, University of Gdansk, Wroclaw University of Technology, Institute of Biotechnology and Molecular Medicine, SensDx S.A

<120> Multifunctional chitosan composite for bone defect filling and bone tissue regeneration and method of obtaining multifunctional chitosan composite for bone defect filling and bone tissue regeneration

<160> SEQ1

<210> 1

<211> 10

<212> ug4

<213> Artificial sequence

<220>

<223> The peptide was designed on the basis of an osteogenic growth peptide and a metalloproteinase-specific sequence and with an additional Cys residue targeting the attachment of the peptide to maleimide-chitosan.

<400>

1                                    10
Cys Pro Leu Gly Leu Tyr Gly Phe Gly Gly

ug1 peptide sequence - SEQ2

<110> Lukasiewicz Research Network - Institute of Ceramics and Building Materials, University of Gdansk, Wroclaw University of Technology, Institute of Biotechnology and Molecular Medicine, SensDx S.A

<120> Multifunctional chitosan composite for bone defect filling and bone tissue regeneration and method of obtaining multifunctional chitosan composite for bone defect filling and bone tissue regeneration

<160> SEQ2

<210> 2

<211> 6

<212> ug1

<213> Artificial sequence

<220>

<223> The peptide was designed based on an osteogenic growth peptide and a metalloproteinase-specific sequence.

<400>

1                          6
Leu Tyr Gly Phe Gly Gly

ug46 peptide sequence - SEQ 3

<110> Lukasiewicz Research Network - Institute of Ceramics and Building Materials, University of Gdansk, Wroclaw University of Technology, Institute of Biotechnology and Molecular Medicine, SensDx S.A

<120> Multifunctional chitosan composite for bone defect filling and bone tissue regeneration and method of obtaining multifunctional chitosan composite for bone defect filling and bone tissue regeneration

<160> SEQ1

<210> 1

<211> 21

<212> ug46

<213> Artificial sequence

<220>

<223> The peptide was designed based on the human cystatin C sequence, amino acid sequence specific for enzymatic cutting by metalloproteases and the antibacterial peptide anoplin.

<400>

1                                                                              21
Ac Gln Ala Gly Ile Val Val Pro Leu Gly Leu Gly Leu Leu Lys Arg Ile Lys Thr Leu Leu

**Claims**

1. A composite with antimicrobial and anti-inflammatory activity for filling bone defects and regenerating bone tissue, in which the matrix is a natural, biodegradable polymer constituted by chitosan, **characterized in that** the chitosan has a deacetylation degree of DD≥72.6%, a viscosity in the range of 351 to 2250 mPas and a molecular weight in the range of 200 to 550 kDa and it represents 24.038 wt.% to 86.256 wt.% of the total composition of a four-component composite, and further contains bioactive glass representing 8.741 wt.% to 74.106 wt.% of the total composition of the four-component composite, produced by the sol- gel method and containing $SiO_2$ in an amount of 60-75 wt.% and CaO in an amount of 10-30 wt.%, and: $P_2O_5$ in an amount of 0-8 wt.%, and/or SrO in an amount of 0-7 wt.%, and/or ZnO in an amount of 0-7 wt.%, and/or CuO in an amount of 0-3 wt.%, and/or MgO in an amount of 0-3 wt.% and has a grain size of 90% less than 250 μm and the composite contains a biologically active, engineered ug4 peptide with an Cys Pro Leu Gly Leu Tyr Gly Phe Gly Gly (SEQ1) sequence representing from 1.130 wt.% to 11.5596 wt.% of the total composition of the four-component composite, wherein the peptide with sequence SEQ1 contains a biologically active peptide with sequence Leu Tyr Gly Phe Gly Gly (SEQ2) and the composite contains a biologically active engineered peptide ug46 with sequence Ac Gln Ala Gly Ile Val Val Pro Leu Gly Leu Gly Leu Leu Lys Arg Ile Lys Thr Leu Leu (SEQ3) representing from 0.044 wt% to 1.575 wt% of the total composition of the composite.

2. A composite with antimicrobial and anti-inflammatory activity for filling bone defects and regenerating bone tissue, in which the matrix is a natural biodegradable polymer constituted by chitosan, **characterised in that** the chitosan has a deacetylation degree of DD≥72.6%, a viscosity in the range of 351 to 2250 mPas and a molecular weight in the range of 200 to 550 kDa and it represents 24.80 wt.% to 89.91 wt.% of the total composition of the composite, and furthermore contains bioactive glass representing from 9.92 wt.% to 74.925 wt.% of the total composition of the composite, produced by the sol-gel method and containing $SiO_2$ in an amount of 60-75 wt.% of the bioglass and CaO in an amount of 10-30 wt.% of the bioglass, and $P_2O_5$ in an amount of 0-8 wt.%, and/or SrO in an amount of 0-7 wt.%, and/or ZnO in an amount of 0-7 wt.%, and/or CuO in an amount of 0-3 wt.%, and/or MgO in an amount of 0-3 wt.%, with a grain size of 90% less than 250 μm, and contains a biologically active engineered peptide with a sequence Ac Gin Ala Gly Ile Val Val Pro Leu Gly Leu Gly Leu Leu Lys Arg Ile Lys Thr Leu Leu (SEQ3) representing 0.1 wt.% to 0.8 wt.% of the total composition of the composite.

3. A method of obtaining the composite according to claim 1, **characterised in that** in the first step a bioglass is prepared by the sol-gel method, the composition of which is in the range of $SiO_2$ in an amount of 60-75 wt.% and CaO in an amount of 10-30 wt.% , and $PO_5$ in an amount of 0-8 wt.%, and/or SrO in an amount of 0-7 wt.%, and/or ZnO in an amount of 0-7 wt.%, and/or CuO in an amount of 0-3 wt.%, and/or MgO in an amount of 0-3 wt.%., and after mixing the bioglass synthesis precursors, the reaction mixture is subjected to drying by raising the temperature from 40 °C to 180 °C, then the dried gel is heated and subjected to grinding to a grain size where 90% of the grains of the resulting bioglass are below 250 μlm, and then the ug4 peptide with the sequence SEQ1 is produced by solid support synthesis by a known method, and this peptide is attached to chitosan by a click chemistry method at a rate of 3.2 g per 3 g of chitosan, which consists in the attachment of the peptide with the sequence SEQ1 to the chitosan modified with maleimidoglycine such way, that the chitosan is suspended in hot water and then stirred until the solution reaches room temperature, while the N-hydroxysuccinimide ester of aminoacetic acid is dissolved in tetrahydrofuran and, after at least 24 hours, the modified chitosan is drained and the washed chitosan is lyophilised, using a covalent bond between the peptide and a maleimide linker attached to the chitosan with a degree of maleimidoglycine modification of 9 to 10%, and in the next step a solution of a mixture of chitosan and chitosan modified with the peptide at a concentration of 0.5-4 wt.% in an acidic aqueous solution is prepared, in which the weight ratio of chitosan to peptide-modified chitosan is from 1.130 to 11.299, and a paste on the basis on bioglass particles wetted with an acidic aqueous solution is prepared with particles concentration ranging from 0.02 g to 0.42 g per 1 ml of solution, after which the resulting paste is introduced into the chitosan solution and mixed at room temperature to produce a stable dispersion of the bioglass particles in the acidic chitosan solution with the peptide in the solution so prepared, then the resulting suspension is lyophilised, wherein the entire system is stabilised before or after the lyophilisation process by chemical or physical crosslinking, and the finished porous structures are washed to neutral pH and freeze-dried again, and then a peptide with the sequence SEQ3 is produced by a known method of synthesis on a carrier, and the peptide is then subjected to a purification process, and, in the final step, an aqueous solution of the peptide obtained is prepared and applied by adsorption to the surface of porous chitosan bioglass structures by immersing the porous scaffold in the peptide solution for 4- 24 hours, and the porous composite is again freeze-dried.

4. A method for obtaining the composite according to claim 2, **characterised in that** in the first step a bioglass is prepared by the sol-gel method, the composition of which is in the range of SiO 2 in an amount of 60-75 wt.% of the bioglass and CaO in an amount of 10-30 wt.% of the bioglass and: P 2 O 5 in an amount of 0-8 wt.%, and/or SrO in an amount of 0-7 wt.%, and/or ZnO in an amount of 0-7 wt.%, and/or CuO in an amount of 0-3 wt.%, and/or MgO in an amount of 0-3 wt.%, and after mixing the bioglass synthesis precursors, the reaction mixture is subjected to slow drying by gradually increasing the temperature from 40 °C to 180 °C and then the dried gel is heated in an electric oven at 500 to 750 °C and subjected to grinding to a grain size in which 90% of the grains of the obtained bioglass are below 250 $\mu$m, and in the next step, a chitosan solution of 0.5-4 wt.% is prepared, in an acidic aqueous solution, and simultaneously, a paste on the basis of bioglass particles of known composition and grain size, wetted with an acidic aqueous solution is prepared, with a particle concentration in the range of 0.02 g to 0.42 g per 1 ml of solution, whereupon the resulting paste is introduced into the chitosan solution and mixed by a known method at room temperature to produce a stable dispersion of the bioglass particles in the acidic chitosan solution, and the resulting suspension is then lyophilised, then the entire chitosan/bioglass system is subjected to stabilisation by chemical or physical crosslinking before or after lyophilisation, and finished porous structures are washed to neutral pH and again freeze-dried, and then a peptide with the sequence SEQ3 is produced by a known carrier synthesis method, and the peptide is then subjected to the process of purification, and in the final step, an aqueous solution of the peptide obtained is prepared and applied by adsorption to the surface of the porous chitosan bioglass structures by immersing the porous scaffold in the peptide solution for 4-24 hours and the porous composite is again freeze-dried.

5. A method for obtaining the composite according to claim 2, **characterised in that** in the first step a bioglass is prepared by the sol-gel method, the composition of which is in the range of $SiO_2$ in an amount of 60-75 wt.% and CaO in an amount of 10-30 wt.%, and: $P_2O_5$ in an amount of 0-8 wt.%, and/or SrO in an amount of 0-7 wt.%, and/or ZnO in an amount of 0-7 wt.%, and/or CuO in an amount of 0-3 wt.%, and/or MgO in an amount of 0-3 wt.%, and after mixing the bioglass synthesis precursors, the reaction mixture is subjected to slow drying by gradually increasing the temperature from 40°C to 180°C and the dried gel is then heated in an electric oven at 500 to 750°C and subjected to grinding to a grain size where 90% of the grains of the resulting bioglass are below 250 $\mu$m, and in the next step, a peptide with the sequence SEQ3 is produced by a known method of synthesis on a solid support, and the peptide is then subjected to a purification process, followed by the production of peptide fibrils "UG46" by thermal incubation in this way, and then a chitosan solution of 0.5-4 wt.% in an acidic aqueous solution is prepared, and at the same time a paste on the basis of bioglass particles of known composition and grain size, wetted with an acidic aqueous solution is prepared, with a particle concentration ranging from 0.02 g to 0.42 g per 1 ml of solution, whereupon the paste produced is introduced into the chitosan solution and mixed at room temperature to produce a stable dispersion of the bioglass particles in the acidic chitosan solution and then a solution of peptide fibrils obtained is introduced into the system and the whole is further mixed until homogeneous, and then the suspension is subjected to lyophilisation, then the entire chitosan/-bioglass/peptide fibrils system is subjected to stabilisation by chemical or physical crosslinking before or after the lyophilisation process, and the finished porous structures of the composite are subjected to washing to neutral pH and are again freeze-dried.

6. The method according to claims 3, 4, wherein the percentage of the active peptide ug46 introduced onto the porous structures by surface adsorption is 0.044-1.575%.

7. The method according to claim 5, wherein the concentration of the aqueous solution of peptide fibrils UG46 introduced into the system is from 0.80 to 0.85 mg/ml.

8. The method according to claim 4, wherein the concentration of peptide ug46 introduced onto the porous structures by surface adsorption is from 0.25 to 0.35 mg/ml.

9. The method according to claims 4, 5, **characterised in that** the peptide ug46 is then subjected to a purification process using reversed phase high performance liquid chromatography.

10. The method according to claims 4, 5 wherein the peptide ug46 is obtained from an SPPS carrier using the Fmoc methodology and the reaction for cleavage of the peptides from the carrier with simultaneous removal of the side chain sheaths is carried out using a reaction mixture comprising trifluoroacetic acid, phenol, water, 1,2-ethanedithiol and triisopropylsilane.

11. The method according to claim 5, wherein the fibrils are obtained so that the ug46 peptide is dissolved in PBS phosphate buffer and the sample is placed in a thermal block and incubated for 7 days at 37°C using continuous shaking at 300 rpm.

**Patentansprüche**

1. Verbundstoff mit antimikrobieller und entzündungshemmender Wirkung zum Füllen von Knochendefekten und zur Regeneration von Knochengewebe, wobei die Matrix ein natürliches, biologisch abbaubares Polymer ist, das aus Chitosan besteht, **dadurch gekennzeichnet, dass** das Chitosan einen Deacetylierungsgrad von DD≥72,6 %, eine Viskosität im Bereich von 351 bis 2250 mPas und ein Molekulargewicht im Bereich von 200 bis 550 kDa aufweist und 24. 038 Gew.-% bis 86,256 Gew.-% der Gesamtzusammensetzung eines Vier-Komponenten-Verbundstoffs ausmacht und ferner bioaktives Glas enthält, das 8,741 Gew.-% bis 74,106 Gew.-% der Gesamtzusammensetzung des Vier-Komponenten-Verbundstoffs ausmacht, durch das Sol-Gel-Verfahren hergestellt wird und SiO2 in einer Menge von 60-75 Gew.-% und CaO in einer Menge von 10-30 Gew.-% enthält, und: P2O5 in einer Menge von 0-8 Gew.-%, und/oder SrO in einer Menge von 0-7 Gew.-%, und/oder ZnO in einer Menge von 0-7 Gew.-%, und/oder CuO in einer Menge von 0-3 Gew.-%, und/oder MgO in einer Menge von 0-3 Gew.-%. % und eine Korngröße von 90 % weniger als 250 $\mu$m aufweist und der Verbundstoff ein biologisch aktives, technisch hergestelltes ug4-Peptid mit einer Cys Pro Leu Gly Leu Tyr Gly Phe Gly Gly (SEQ1)-Sequenz enthält, die von 1,130 Gew.-% bis 11,5596 Gew. % der Gesamtzusammensetzung des Vier-Komponenten-Verbundstoffs ausmacht, wobei das Peptid mit der Sequenz SEQ1 ein biologisch aktives Peptid mit der Sequenz Leu Tyr Gly Phe Gly Gly (SEQ2) enthält und der Verbundstoff ein biologisch aktives, technisch hergestelltes Peptid ug46 mit der Sequenz Ac Gln Ala Gly Ile Val Val Pro Leu Gly Leu Gly Leu Leu Leu Lys Arg Ile Lys Thr Leu Leu (SEQ3) enthält, das 0,044 Gew.-% bis 1,575 Gew.-% der Gesamtzusammensetzung des Verbundstoffs ausmacht.

2. Verbundstoff mit antimikrobieller und entzündungshemmender Wirkung zum Füllen von Knochendefekten und zur Regeneration von Knochengewebe, wobei die Matrix ein natürliches, biologisch abbaubares Polymer ist, das aus Chitosan besteht, **dadurch gekennzeichnet, dass** das Chitosan einen Deacetylierungsgrad von DD≥72. 6 %, eine Viskosität im Bereich von 351 bis 2250 mPas und ein Molekulargewicht im Bereich von 200 bis 550 kDa aufweist und 24,80 Gew.-% bis 89,91 Gew.-% der Gesamtzusammensetzung des Verbundstoffs ausmacht und außerdem bioaktives Glas enthält, das 9,92 Gew.-% bis 74,925 Gew.-% der Gesamtzusammensetzung des Verbundstoffs ausmacht, durch das Sol-Gel-Verfahren hergestellt ist und SiO2 in einer Menge von 60-75 Gew.-% des Bioglases und CaO in einer Menge von 10-30 Gew.-% des Bioglases und P2O5 in einer Menge von 0-8 Gew.-% und/oder SrO in einer Menge von 0-7 Gew.-% und/oder ZnO in einer Menge von 0-7 Gew.-% und/oder CuO in einer Menge von 0-3 Gew.-% und/oder MgO in einer Menge von 0-3 Gew.-%, mit einer Korngröße von 90 % weniger als 250 $\mu$m enthält, und: enthält ein biologisch aktives, technisch hergestelltes Peptid mit einer Sequenz Ac Gln Ala Gly Ile Val Val Pro Leu Gly Leu Gly Leu Leu Lys Arg Ile Lys Thr Leu Leu (SEQ3), die 0,1 Gew.-% bis 0,8 Gew.-% der Gesamtzusammensetzung des Verbundstoffs ausmacht.

3. Verfahren zur Herstellung des Verbundwerkstoffs nach Anspruch 1, **dadurch gekennzeichnet, dass** in der ersten Stufe ein Bioglas nach dem Sol-Gel-Verfahren hergestellt wird, dessen Zusammensetzung im Bereich von SiO2 in einer Menge von 60-75 Gew. % und CaO in einer Menge von 10-30 Gew.% und PO5 in einer Menge von 0-8 Gew.% und/oder SrO in einer Menge von 0-7 Gew.% und/oder ZnO in einer Menge von 0-7 Gew.% und/oder CuO in einer Menge von 0-3 Gew.% und/oder MgO in einer Menge von 0-3 Gew.% liegt und nach dem Mischen der Vorläufer für die Bioglassynthese wird. Das Reaktionsgemisch wird einer Trocknung durch Erhöhen der Temperatur von 40 °C auf 180 °C unterzogen, dann wird das getrocknete Gel erhitzt und einer Vermahlung auf eine Korngröße unterzogen, bei der 90 % der Körner des resultierenden Bioglases unter 250 $\mu$lm liegen, und dann wird das ug4-Peptid mit der Sequenz SEQ1 durch Festträgersynthese nach einem bekannten Verfahren hergestellt, und dieses Peptid wird durch ein Click-Chemie-Verfahren mit einer Rate von 3. 2 g pro 3 g Chitosan an Chitosan gebunden, das darin besteht, dass das Peptid mit der Sequenz SEQ1 an das mit Maleimidoglycin modifizierte Chitosan derart gebunden wird, dass das Chitosan in heißem Wasser suspendiert und dann gerührt wird, bis die Lösung Raumtemperatur erreicht, während der N-Hydroxysuccinimid-Ester der Aminoessigsäure in Tetrahydrofuran gelöst wird und, nach mindestens 24 Stunden wird das modifizierte Chitosan entwässert und das gewaschene Chitosan lyophilisiert, wobei eine kovalente Bindung zwischen dem Peptid und einem an das Chitosan gebundenen Maleimid-Linker mit einem Maleimidoglycin-Modifizierungsgrad von 9 bis 10 % verwendet wird, und im nächsten Schritt eine Lösung einer Mischung aus Chitosan und mit dem Peptid modifiziertem Chitosan in einer Konzentration von 0. 5-4 Gew.-% in einer sauren wässrigen Lösung hergestellt wird, wobei das Gewichtsverhältnis von Chitosan zu peptidmodifiziertem Chitosan 1,130 bis 11,299 beträgt, und eine Paste auf der Basis von Bioglaspartikeln, die mit einer sauren wässrigen Lösung benetzt sind, mit einer Partikelkonzentration von 0,02 g bis 0. 42 g pro 1 ml Lösung hergestellt wird, wonach die resultierende Paste in die Chitosanlösung eingebracht und bei Raumtemperatur gemischt wird, um eine stabile Dispersion der Bioglaspartikel in der sauren Chitosanlösung mit dem Peptid in der so hergestellten Lösung herzustellen, dann die resultierende Suspension gefriergetrocknet wird, wobei das gesamte System vor oder nach dem Gefriertrocknungsprozess durch chemische oder physikalische Vernetzung stabilisiert wird, und die fertigen porösen Strukturen auf neutralen pH-Wert gewaschen und wieder gefriergetrocknet werden, und anschließend ein Peptid mit der Sequenz SEQ3 durch ein bekanntes Syntheseverfahren auf einem Träger hergestellt und das Peptid anschließend einem Reinigungsverfahren unterzogen wird und im letzten Schritt eine wässrige Lösung des erhaltenen Peptids hergestellt und durch Adsorption auf die Oberfläche von porösen Chitosan-Bioglas-Strukturen aufgebracht wird, indem das poröse Gerüst für 4 bis 24 Stunden in die Peptidlösung getaucht wird, und der poröse Verbund erneut gefriergetrocknet wird.

4. Verfahren zur Herstellung des Verbundwerkstoffs nach Anspruch 2, **dadurch gekennzeichnet, dass** in der ersten Stufe ein Bioglas nach dem Sol-Gel-Verfahren hergestellt wird, dessen Zusammensetzung im Bereich von SiO$_2$ in einer Menge von 60-75 Gew.-% des Bioglases und CaO in einer Menge von 10-30 Gew.-% des Bioglases liegt: P$_2$O$_5$ in einer Menge von 0-8 Gew.-% und/oder SrO in einer Menge von 0-7 Gew.-% und/oder ZnO in einer Menge von 0-7 Gew.-% und/oder CuO in einer Menge von 0-3 Gew.-% und/oder MgO in einer Menge von 0-3 Gew.-%. %, und nach dem Mischen der Vorläufer für die Bioglassynthese wird das Reaktionsgemisch einer langsamen Trocknung unterzogen, indem die Temperatur allmählich von 40 °C auf 180 °C erhöht wird, und dann wird das getrocknete Gel in einem elektrischen Ofen auf 500 bis 750 °C erhitzt und einem Mahlen auf eine Korngröße unterzogen, bei der 90 % der Körner des erhaltenen Bioglases unter 250 $\mu$m liegen, und im nächsten Schritt wird eine Chitosanlösung von 0. 5-4 Gew.-% in einer sauren wässrigen Lösung hergestellt, und gleichzeitig wird eine Paste auf der Basis von Bioglaspartikeln bekannter Zusammensetzung und Korngröße, die mit einer sauren wässrigen Lösung benetzt sind, mit einer Partikelkonzentration im Bereich von 0,02 g bis 0. 42 g pro 1 ml Lösung hergestellt, woraufhin die resultierende Paste in die Chitosanlösung eingebracht und nach einem bekannten Verfahren bei Raumtemperatur gemischt wird, um eine stabile Dispersion der Bioglaspartikel in der sauren Chitosanlösung herzustellen, und die resultierende Suspension anschließend gefriergetrocknet wird, dann das gesamte Chitosan/Bioglassystem vor oder nach der Gefriertrocknung einer Stabilisierung durch chemische oder physikalische Vernetzung unterzogen wird und die fertigen porösen Strukturen auf neutralen pH gewaschen und erneut gefriergetrocknet werden, und dann ein Peptid mit der Sequenz SEQ3 durch eine bekannte Trägersynthesemethode hergestellt wird, und das Peptid dann dem Prozess der Reinigung unterzogen wird, und im letzten Schritt eine wässrige Lösung des erhaltenen Peptids hergestellt und durch Adsorption auf die Oberfläche der porösen Chitosan-Bioglas-Strukturen aufgebracht wird, indem das poröse Gerüst für 4-24 Stunden in die Peptidlösung getaucht wird und der poröse Verbundstoff erneut gefriergetrocknet wird.

5. Verfahren zur Herstellung des Verbundwerkstoffs nach Anspruch 2, **dadurch gekennzeichnet, dass** in der ersten Stufe ein Bioglas nach dem Sol-Gel-Verfahren hergestellt wird, dessen Zusammensetzung im Bereich von SiO2 in einer Menge von 60-75 Gew.-% und CaO in einer Menge von 10-30 Gew.-% liegt, und: P2O5 in einer Menge von 0-8 Gew.-% und/oder SrO in einer Menge von 0-7 Gew.-% und/oder ZnO in einer Menge von 0-7 Gew.-% und/oder CuO in einer Menge von 0-3 Gew.-% und/oder MgO in einer Menge von 0-3 Gew.-%. %, und nach dem Mischen der Vorläufer für die Bioglassynthese wird das Reaktionsgemisch einer langsamen Trocknung unterzogen, indem die Temperatur allmählich von 40°C auf 180°C erhöht wird, und das getrocknete Gel wird dann in einem elektrischen Ofen auf 500 bis 750°C erhitzt und einem Mahlen auf eine Korngröße unterzogen, bei der 90% der Körner des resultierenden Bioglases unter 250 $\mu$m liegen, und im nächsten Schritt ein Peptid mit der Sequenz SEQ3 durch ein bekanntes Syntheseverfahren auf einem festen Träger hergestellt wird, und das Peptid dann einem Reinigungsverfahren unterzogen wird, gefolgt von der Herstellung von Peptidfibrillen "UG46" durch thermische Inkubation auf diese Weise, und dann eine Chitosanlösung von 0. 5-4 Gew. % in einer sauren wässrigen Lösung hergestellt, und gleichzeitig wird eine Paste auf der Basis von Bioglaspartikeln bekannter Zusammensetzung und Korngröße, die mit einer sauren wässrigen Lösung benetzt sind, mit einer Partikelkonzentration von 0,02 g bis 0. 42 g pro 1 ml Lösung hergestellt, woraufhin die hergestellte Paste in die Chitosanlösung eingebracht und bei Raumtemperatur gemischt wird, um eine stabile Dispersion der Bioglaspartikel in der sauren Chitosanlösung herzustellen, und anschließend eine Lösung der erhaltenen Peptidfibrillen in das System eingebracht und das Ganze weiter gemischt wird, bis es homogen ist, und dann wird die Suspension einer Gefriertrocknung unterzogen, dann wird das gesamte System Chitosan/Bioglas/Peptidfibrillen einer Stabilisierung durch chemische oder physikalische Vernetzung vor oder nach dem Gefriertrocknungsprozess unterzogen, und die fertigen porösen Strukturen des Komposits werden einer Wäsche auf neutralen pH-Wert unterzogen und erneut gefriergetrocknet.

6. Verfahren nach Anspruch 3 oder 4, wobei der Prozentsatz des aktiven Peptids ug46, das durch Oberflächenadsorption in die porösen Strukturen eingebracht wird, 0,044-1,575% beträgt.

7. Verfahren nach Anspruch 5, wobei die Konzentration der wässrigen Lösung von Peptidfibrillen UG46, die in das System eingebracht wird, von 0,80 bis 0,85 mg/ml beträgt.

**8.** Verfahren nach Anspruch 4, wobei die Konzentration des Peptids UG46, das durch Oberflächenadsorption in die porösen Strukturen eingebracht wird, 0,25 bis 0,35 mg/ml beträgt.

**9.** Verfahren nach Anspruch 4 oder 5, **dadurch gekennzeichnet, dass** das Peptid ug46 anschließend einem Reinigungsverfahren unter Verwendung von Umkehrphasen-Hochleistungsflüssigkeitschromatographie unterzogen wird.

**10.** Verfahren nach Anspruch 4 oder 5, **dadurch gekennzeichnet, dass** das Peptid ug46 aus einem SPPS-Träger unter Verwendung der Fmoc-Methode gewonnen wird und die Reaktion zur Abspaltung der Peptide vom Träger bei gleichzeitiger Entfernung der Seitenkettenhüllen unter Verwendung einer Reaktionsmischung aus Trifluoressigsäure, Phenol, Wasser, 1,2-Ethandithiol und Triisopropylsilan durchgeführt wird.

**11.** Verfahren nach Anspruch 5, wobei die Fibrillen so erhalten werden, dass das ug46-Peptid in PBS-Phosphatpuffer gelöst und die Probe in einen Thermoblock gegeben und 7 Tage lang bei 37°C unter kontinuierlichem Schütteln bei 300 U/min inkubiert wird.

**Revendications**

**1.** Le composite à activité antimicrobienne et anti-inflammatoire pour combler les défauts osseux et régénérer le tissu osseux, dans lequel la matrice est un polymère naturel biodégradable constitué par du chitosane, **caractérisé en ce que** le chitosane a un degré de désacétylation de DD≥72,6 %, une viscosité comprise entre 351 et 2250 mPas et un poids moléculaire compris entre 200 et 550 kDa. Il représente 24,038 % en poids à 86,256 % en poids de la composition totale du composite à quatre composants, et contient en outre du verre bioactif représentant de 8,741 % en poids à 74,106 % en poids de la composition totale du composite à quatre composants, produit par le procédé sol-gel et contenant $SiO_2$ dans une quantité de 60-75 % en poids et CaO dans une quantité de 10-30 % en poids, et : $P_2O_5$ dans une proportion de 0-8 % en poids, et/ou SrO dans une proportion de 0-7 % en poids, et/ou ZnO dans une proportion de 0-7 % en poids, et/ou CuO dans une proportion de 0-3 % en poids, et/ou MgO dans une proportion de 0-3 % en poids, et dont 90% a une granulométrie inférieure à 250 $\mu$m. Le composite contient un peptide ug4 biologiquement actif conçu avec une séquence Cys Pro Leu Gly Leu Tyr Gly Phe Gly Gly (SEQ1) représentant de 1,130 % en poids à 11,5596 % en poids de la composition totale du composite à quatre composants, dans lequel le peptide avec la séquence SEQ1 contient un peptide bioactif avec la séquence Leu Tyr Gly Phe Gly Gly (SEQ2) et le composite contient un peptide biologiquement actif conçu ug46 avec la séquence Ac Gln Ala Gly Ile Val Val Pro Leu Gly Leu Gly Leu Lys Arg Ile Lys Thr Leu Leu (SEQ3) représentant de 0,044 % en poids à 1,575 % en poids de la composition totale du composite.

**2.** Le composite à activité antimicrobienne et anti-inflammatoire destiné à combler les défauts osseux et à régénérer le tissu osseux, dans lequel la matrice est un polymère naturel biodégradable constitué de chitosane, **caractérisé par le fait que** le chitosane a un degré de désacétylation de DD≥72. 6 %, une viscosité comprise entre 351 et 2250 mPas et un poids moléculaire compris entre 200 et 550 kDa. Il représente de 24,80 % en poids à 89,91 % en poids de la composition totale du composite et contient en outre du verre bioactif représentant de 9,92 % en poids à 74,925 % en poids de la composition totale du composite, produit-par le procédé sol-gel et contenant $SiO_2$ dans une proportion de 60-75 % en poids du verre biologique et CaO dans une proportion de 10-30 % en poids du verre bioactif, et $P_2O_5$ dans une proportion de 0-8 % en poids, et/ou SrO dans une proportion de 0-7 % en poids, et/ou ZnO dans une proportion de 0-7 % en poids, et/ou CuO dans une proportion de 0-3 % en poids, et/ou MgO dans une proportion de 0-3 % en poids, dont 90% a une granulométrie inférieure à 250 $\mu$m, et contient un peptide bioactif conçu avec la séquence Ac Gln Ala Gly Ile Val Val Pro Leu Gly Leu Gly Leu Leu Lys Arg Ile Lys Thr Leu Leu (SEQ3) représentant de 0,1% en poids à 0,8% en poids de la composition totale du composite.

**3.** La méthode d'obtention du composite selon la revendication 1, est **caractérisée par le fait que** dans la première étape, on prépare le verre bioactif par le procédé sol-gel, dont la composition se situe dans la gamme suivante : $SiO_2$ dans une quantité de 60-75 % en poids et CaO dans une proportion de 10-30 % en poids, et $P_2O_5$ dans une proportion de 0-8 % en poids, et/ou SrO dans une proportion de 0-7 % en poids, et/ou ZnO dans une proportion de 0-7 % en poids, et/ou CuO dans une proportion de 0-3 % en poids, et/ou MgO dans une proportion de 0-3 % en poids. Après avoir mélangé les précurseurs de la synthèse de verre bioactif, le mélange réactionnel est soumis à un séchage en augmentant la température de 40 °C à 180 °C, puis le gel séché est chauffé et soumis à un broyage pour obtenir la granulométrie dans laquelle 90% des particules du verre bioactif obtenu sont inférieures à 250 $\mu$lm. Puis le peptide ug4 avec la séquence SEQ1 est produit par synthèse sur support solide selon une méthode connue, et ce peptide se lie au chitosane par une méthode de chimie click à une quantité de 3, 2 g pour 3 g de chitosane, qui consiste à lier le peptide avec la séquence SEQ1 au chitosane modifié avec de la maléimidoglycine de telle manière que le chitosane est suspendu dans de l'eau chaude et ensuite agité jusqu'à ce que la solution atteigne la température ambiante, tandis que l'ester N-hydroxysuccinimide de l'acide aminoacétique est dissous dans du tétrahydrofurane et, après au moins 24 heures, le chitosane modifié est égoutté et le chitosane lavé est lyophilisé, en utilisant une liaison covalente entre le peptide et un lien maléimide lié au chitosane avec un degré de modification de la maléimidoglycine de 9 à 10%. Dans l'étape suivante, on prépare une solution d'un mélange de chitosane et de chitosane modifié par le peptide à une concentration de 0,5-4 % en poids dans une solution aqueuse acide, dans laquelle le rapport en poids entre le chitosane et le chitosane modifié par le peptide est compris entre 1,130 et 11,299 et on prépare une pâte à base des particules de verre bioactif humidifiées avec la solution aqueuse acide ayant une concentration de particules de 0,02g à 0,42g par 1 ml de solution. Ensuite la pâte résultante est introduite dans la solution de chitosane et mélangée à température ambiante pour produire une dispersion stable des particules de verre bioactif dans la solution acide de chitosane avec le peptide dans la solution ainsi préparée. Ensuite la suspension résultante est lyophilisée, l'ensemble du système étant stabilisé avant ou après le processus de lyophilisation par réticulation chimique ou physique, et les structures poreuses finies sont lavées jusqu'à un pH neutre et lyophilisées à nouveau, puis un peptide de la séquence SEQ3 est produit par une méthode de synthèse connue sur un support, et le peptide est ensuite soumis à un processus de purification, et, dans l'étape finale, on prépare une solution aqueuse du peptide obtenu et on l'applique par adsorption à la surface de structures poreuses de chitosane et verre bioactif en immergeant l'échafaudage poreux dans la solution de peptide pendant 4 à 24 heures, et le composite poreux est à nouveau lyophilisé.

4. La méthode d'obtention du composite selon la revendication 2, est **caractérisée par le fait que** dans la première étape, on prépare le verre bioactif par le procédé sol-gel, dont la composition se situe dans la gamme suivante $SiO_2$ dans une quantité de 60-75 % en poids du verre bioactif et CaO dans une quantité de 10-30 % en poids du verre bioactif, et : $P_2O_5$ dans une quantité de 0-8 % en poids, et/ou SrO dans une quantité de 0-7 % en poids, et/ou ZnO dans une quantité de 0-7 % en poids, et/ou CuO dans une quantité de 0-3 % en poids et/ou MgO dans une proportion de 0-3 % en poids, et après avoir mélangé les précurseurs de synthèse de verre bioactif, le mélange réactionnel est soumis à un chauffage lent en augmentant progressivement la température de 40 °C à 180 °C, puis le gel séché est chauffé dans un four entre 500 et 750 °C et soumis à un broyage pour obtenir la granulométrie dans laquelle 90% des particules du verre bioactif obtenu sont inférieures à 250 $\mu$m. Dans l'étape suivante, on prépare une solution de chitosane de concentration de 0,5 à 4 % en poids dans une solution aqueuse acide et, simultanément, on prépare une pâte à base de particules de verre bioactif de composition et de granulométrie connues, mouillées avec une solution aqueuse acide, avec une concentration de particules comprise entre 0,02 g et 0,42 g par 1 ml de solution. Ensuite la pâte obtenue est introduite dans la solution de chitosane et mélangée selon une méthode connue à température ambiante pour produire une dispersion stable des particules de verre bioactif dans la solution acide de chitosane, et puis la suspension résultante est ensuite lyophilisée, l'ensemble du système chitosane/verre bioactif est ensuite soumis à une stabilisation par réticulation chimique ou physique avant ou après la lyophilisation, et les structures poreuses finies sont lavées jusqu'à un pH neutre et à nouveau lyophilisées. Ensuite on produit un peptide avec la séquence SEQ3 par une méthode de synthèse de support connue. Le peptide est ensuite soumis au processus de purification et, dans l'étape finale, on prépare une solution aqueuse du peptide obtenu et on l'applique par adsorption à la surface des structures poreuses en chitosane et en verre bioactif en immergeant l'échafaudage poreux dans la solution de peptide pendant 4 à 24 heures, et le composite poreux est à nouveau lyophilisé.

5. La méthode d'obtention du composite selon la revendication 2, est **caractérisée par le fait que** dans la première étape, on prépare un verre bioactif par le procédé sol-gel, dont la composition se situe dans la gamme suivante : $SiO_2$ dans une proportion de 60-75 % en poids et CaO dans une proportion de 10-30 % en poids, et : $P_2O_5$ dans une proportion de 0-8 % en poids, et/ou SrO dans une proportion de 0-7 % en poids, et/ou ZnO dans une proportion de 0-7 % en poids, et/ou CuO dans une proportion de 0-3 % en poids, et/ou MgO dans une proportion de 0-3 % en poids, et après avoir mélangé les précurseurs de la synthèse de verre bioactif, le mélange réactionnel est soumis à un chauffage lent en augmentant progressivement la température de 40°C à 180°C et le gel séché est ensuite chauffé dans un four entre 500 et 750°C et soumis à un broyage pour obtenir la granulométrie dans laquelle 90% des particules du verre bioactif obtenu sont inférieures à 250 $\mu$m. Dans l'étape suivante, on produit un peptide avec la séquence SEQ3 par une méthode connue de synthèse sur un support solide, et le peptide est ensuite soumis à un processus de purification, suivi par la production de fibrilles peptidiques "UG46" par incubation thermique de cette manière. On prépare ensuite une solution de chitosane de concentration de 0,5 à 4 % en poids dans une solution aqueuse acide et, en même temps, on prépare une pâte à base de particules de verre bioactif de composition et de granulométrie données, mouillées avec une solution aqueuse acide, avec une concentration de particules allant de 0,02 g à 0,42 g par 1 ml de solution. Ensuite, la pâte produite est introduite dans la solution de chitosane et mélangée à température ambiante pour produire une dispersion stable des particules de verre bioactif dans la solution acide de chitosane, puis une solution de fibrilles peptidiques est introduite dans le système et l'ensemble est mélangé jusqu'à obtention d'une solution homogène. La suspension est ensuite soumise à la lyophilisation, puis l'ensemble du système chitosane/verre bioactif /fibrilles peptidiques est soumis à une stabilisation par réticulation chimique ou physique avant ou après le processus de lyophilisation, et les structures poreuses finies du composite sont soumises à un lavage jusqu'à un pH neutre et sont à nouveau lyophilisées.

6. La méthode selon les revendications 3 et 4, est **caractérisée par le fait que** le pourcentage du peptide actif ug46 introduit dans les structures poreuses par adsorption de surface est compris entre 0,044 et 1,575 %.

7. La méthode selon la revendication 5, est **caractérisée par le fait que** la concentration de la solution aqueuse de fibrilles peptidiques UG46 introduite dans le système est de 0,80 à 0,85 mg/ml.

8. La méthode selon la revendication 4, est **caractérisée par le fait que** la concentration du peptide ug46 introduit sur les structures poreuses par adsorption de surface est de 0,25 à 0,35 mg/ml.

9. La méthode selon les revendications 4 et 5, est **caractérisée par le fait que** le peptide ug46 est ensuite soumis à un processus de purification par chromatographie liquide haute performance en phase inversée.

10. La méthode selon les revendications 4 et 5, est **caractérisée par le fait que** le peptide ug46 est obtenu à partir d'un support SPPS en utilisant la méthodologie Fmoc et la réaction de clivage des peptides du support avec élimination simultanée des protections des chaînes latérales est effectuée en utilisant un mélange réactionnel comprenant de l'acide trifluoroacétique, du phénol, de l'eau, du 1,2-éthanedithiol et du triisopropylsilane.

11. La méthode selon la revendication 5, est **caractérisée par le fait que** les fibrilles sont obtenues de telle sorte que le peptide ug46 est dissous dans une solution tampon phosphate PBS et l'échantillon est placé dans un bloc thermique et incubé pendant 7 jours à 37°C en utilisant une agitation continue à 300 rpm.

Fig.1.

Fig. 2.

metalloproteinase cleavage sites

ug4           ug1

C P L G **L Y G F G G**    (modified growth factor fragment)

connection of maleimide to peptide via cysteine

Gly

CHITOSAN

Fig. 3.

Fig.4.

Fig. 5.

Fig.6.

Fig 7.

Fig. 8.

Fig. 9.

Fig. 10.

Fig. 11.

Fig. 12.

Fig. 13.

Fig. 14.

Fig. 15.

Fig. 16.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 8778378 B
- US 5977204 A
- US 11225430 B
- US 9801946 B
- US 4895724 A
- US 8853165 B2
- US 20170014473 A1
- WO 2022084288 A1
- US 8546529 B2

**Non-patent literature cited in the description**

- Bone grafts: which is the ideal biomaterial?. *J. Clin. Periodontol.*, 2019, vol. 46, 92-102
- Design of artificial extracellular matrices for tissue engineering. *Prog Polym Sci.*, 2011, vol. 36, 238-268
- Mesoporous bioactive glasses: Promising platforms for antibacterial strategies. *Acta Biomat.*, 2018, vol. 81, 1-19
- *Int. J. Appl. Ceram. Techn.*, 2017, vol. 14, 1107-16
- Bioactivity and osteoinductivity of glasses and glassceramics and their material determinants. *Ceram. Int.*, 2016, vol. 42, 14313-25
- A Review of Bioactive Glass/Natural Polymer Composites: State of the Art. *Mater*, 2020, vol. 13, 1-38
- Polymeric biomaterials. *Acta Mater.*, 2000, vol. 48, 263-277
- Chitosan: A versatile biopolymer for orthopaedic tissue- engineering. *Biomaterials*, 2005, vol. 26, 5983-5990
- Prospects of chitosan-based scaffolds for growth factor release in tissue engineering. *Int J Biol Macromol*, 2016, vol. 93, 1382-1389
- 3D Nanocomposite chitosan/bioactive glass scaffolds obtained using two different routes: an evaluation of the porous structure and mechanical properties. *Quim. Nova*, 2016, vol. 39 (4), 462-466
- Molecular Control of Bioactivity in Sol-Gel Glasses. *J. Sol-Gel Sci. Tech.*, 1998, vol. 13, 245-50
- The synergistic effects of Sr and Si bioactive ions on osteogenesis, osteoclastogenesis and angiogenesis for osteoporotic bone regeneration. *Acta Biomat.*, 2017, vol. 61, 217-232
- The effects of strontium-substituted bioactive glasses on osteoblasts and osteoclasts in vitro. *Biomat.*, 2010, vol. 31, 3949-56
- Graphene oxide, chitosan and silver nanocomposite as a highly effective antibacterial agent against pathogenic strains. *Coll. Surfaces A*, 2018, vol. 13, 245-50
- Glasses in bone regeneration: a multiscale issue. *J. Non-Cryst. Solids*, 2016, vol. 432, 9-14
- Mesoporous silica-based bioactive glasses for antibiotic-free antibacterial applications. *Mater. Sci. Eng. C*, 2018, vol. 83, 99-107
- Preparation and characterisation of trace elements-multidoped injectable biomimetic materials for minimally invasive treatment of osteoporotic bone trauma. *J Biomed Mat Res A*, 2010, vol. 95A (4), 1170-81
- *Society for Biomaterials*, 2017, vol. 105 (7), 1845-1855
- *Int J Appl Ceram Technol.*, 2017, vol. 14, 1107-1116
- *Int. J. Appl. Ceram. Techn.*, 2020, vol. 17, 2807-16
- *Advances in Colloid and Interface Science*, 2020, vol. 276, 102083
- Lukasiewicz Research Network - Institute of Ceramics and Building Materials. University of Gdansk, Wroclaw University of Technology, Institute of Biotechnology and Molecular Medicine
- Lukasiewicz Research Network. Institute of Ceramics and Building Materials, University of Gdansk, Wroclaw University of Technology, Institute of Biotechnology and Molecular Medicine